# EUROPEAN PATENT SPECIFICATION

(11) **EP 3 990 667 B1**
(45) Date of publication and mention of the grant of the patent: **31.01.2024**
(21) Application number: 21709989.4
(22) Date of filing: 05.03.2021
(51) Int. Cl.: C12Q 1/70

(54) **METHODS FOR THE SPECIFIC AND SENSITIVE DETECTION OF SARS-COV-2**
VERFAHREN ZUM SPEZIFISCHEN UND SENSITIVEN NACHWEIS VON SARS-COV-2
PROCÉDÉS POUR LA DÉTECTION SPÉCIFIQUE ET SENSIBLE DU SRAS-COV-2

(30) Priority: 05.03.2020 WO PCT/EP2020/055939; 05.03.2020 US 202016809717
(43) Date of publication of application: 04.05.2022
(73) Proprietor: Institut Pasteur, 75724 Paris Cédex 15 (FR)
(72) Inventor: ENOUF, Vincent, 75724 Paris (FR); DONATI, Flora, 75724 Paris (FR); ALBERT, Mélanie, 75724 Paris (FR); BEHILLIL, Sylvie, 75724 Paris (FR); VAN DER WERF, Sylvie, 75724 Paris (FR); SIMON-LORIERE, Etienne, 75724 Paris (FR); LEMOINE, Frédéric, 75724 Paris (FR); MALABAT, Christophe, 75724 Paris (FR); MARCHIO, Agnés, 75724 Paris (FR); PINEAU, Pascal, 75724 Paris (FR)
(74) Representative: Plasseraud IP
(86) International application number: PCT/EP2021/055695
(87) International publication number: WO 2021/176099

(56) References cited:
- CN-A- 111 394 522
- Who Team: "Molecular assays to diagnose COVID-19: Summary table of available protocols", , 24 January 2020 (2020-01-24), XP055732018, Retrieved from the Internet: URL:https://www.who.int/docs/default-sourc e/coronaviruse/whoinhouseassays.pdf?sfvrsn =de3a76aa_2&download=true [retrieved on 2020-09-18]
- VICTOR M CORMAN ET AL: "Detection of 2019 novel coronavirus (2019-nCoV) by real-time RT-PCR", EUROSURVEILLANCE, vol. 25, no. 3, 23 January 2020 (2020-01-23), XP055695049, FR ISSN: 1560-7917, DOI: 10.2807/1560-7917.ES.2020.25.3.2000045
- WU FAN ET AL: "A new coronavirus associated with human respiratory disease in China", NATURE, MACMILLAN JOURNALS LTD., ETC, LONDON, vol. 579, no. 7798, 3 February 2020 (2020-02-03), pages 265-269, XP037099569, ISSN: 0028-0836, DOI: 10.1038/S41586-020-2008-3 [retrieved on 2020-02-03]
- N N: "Investigation of novel SARS-COV-2 variant Variant of Concern 202012/01 Investigation of novel SARS-COV-2 variant", , 21 December 2020 (2020-12-21), pages 1-11, XP055803098, Retrieved from the Internet: URL:https://assets.publishing.service.gov. uk/government/uploads/system/uploads/attac hment_data/file/959438/Technical_Briefing_ VOC_SH_NJL2_SH2.pdf [retrieved on 2021-05-10]
- Tegally Houriiyah ET AL: "Summary", medRxiv, 22 December 2020 (2020-12-22), XP055803107, DOI: 10.1101/2020.12.21.20248640 Retrieved from the Internet: URL:https://www.medrxiv.org/content/10.110 1/2020.12.21.20248640v1.full.pdf [retrieved on 2021-05-10]
- Voloch Carolina M ET AL: "Article Summary Line", medRxiv, 25 December 2020 (2020-12-25), pages 1-31, XP055803117, DOI: 10.1101/2020.12.23.20248598 Retrieved from the Internet: URL:https://www.medrxiv.org/content/10.110 1/2020.12.23.20248598v1 [retrieved on 2021-05-10]
- Lopez-Rincon Alejandro ET AL: "ABSTRACT", bioRxiv, 21 January 2021 (2021-01-21), pages 1-16, XP055803124, DOI: 10.1101/2021.01.20.427043 Retrieved from the Internet: URL:https://www.biorxiv.org/content/10.110 1/2021.01.20.427043v1.full.pdf [retrieved on 2021-05-10]
- WEI FENG ET AL: "Molecular Diagnosis of COVID-19: Challenges and Research Needs", ANALYTICAL CHEMISTRY, vol. 92, no. 15, 23 June 2020 (2020-06-23) , pages 10196-10209, XP055760336, US ISSN: 0003-2700, DOI: 10.1021/acs.analchem.0c02060
- Rambaut Andrew ET AL: "Preliminary genomic characterisation of an emergent SARS-CoV-2 lineage in the UK defined by a novel set of spike mutations - SARS-CoV-2 coronavirus / nCoV-2019 Genomic Epidemiology - Virological", , 1 December 2020 (2020-12-01), XP055802562, Retrieved from the Internet: URL:https://virological.org/t/preliminary- genomic-characterisation-of-an-emergent-sa rs-cov-2-lineage-in-the-uk-defined-by-a-no vel-set-of-spike-mutations/563 [retrieved on 2021-05-07]
- Tegally Houriiyah ET AL: "Emergence and rapid spread of a new severe acute respiratory syndrome-related coronavirus 2 (SARS-CoV-2) lineage with multiple spike mutations in South Africa", medRxiv, 22 December 2020 (2020-12-22), XP055926915, DOI: 10.1101/2020.12.21.20248640 Retrieved from the Internet: URL:https://www.medrxiv.org/content/10.110 1/2020.12.21.20248640v1.full.pdf [retrieved on 2022-06-01]
- Faria Nuno R. ET AL: "Genomic characterisation of an emergent SARS-CoV-2 lineage in Manaus: preliminary findings - SARS-CoV-2 coronavirus / nCoV-2019 Genomic Epidemiology - Virological", , 13 January 2021 (2021-01-13), XP055809063, Retrieved from the Internet: URL:https://virological.org/t/genomic-char acterisation-of-an-emergent-sars-cov-2-lin eage-in-manaus-preliminary-findings/586 [retrieved on 2021-05-31]
- Wang Pengfei ET AL: "Increased Resistance of SARS-CoV-2 Variants B.1.351 and B.1.1.7 to Antibody Neutralization", bioRxiv, 26 January 2021 (2021-01-26), XP055854285, United States DOI: 10.1101/2021.01.25.428137 Retrieved from the Internet: URL:https://www.biorxiv.org/content/10.110 1/2021.01.25.428137v2.full.pdf [retrieved on 2021-10-25]
- Wang Pengfei ET AL: "Increased Resistance of SARS-CoV-2 Variant P.1 to Antibody Neutralization", bioRxiv, 2 March 2021 (2021-03-02), XP055973984, DOI: 10.1101/2021.03.01.433466 Retrieved from the Internet: URL:https://www.biorxiv.org/content/10.110 1/2021.03.01.433466v1.full.pdf [retrieved on 2022-10-24]

## Description

### FIELD OF THE INVENTION

The invention relates to a method to detect the presence or absence of variants of the 2019 novel coronavirus (SARS-CoV-2, 2019-nCov or COVID-19) in a sample as defined in the claims. The method is useful for screening samples for presence or absence of SARS-CoV-2 variants and may therefore be used to screen subjects to identify individuals infected with SARS-CoV-2 variants, among other uses.

### BACKGROUND OF THE INVENTION

In December 2019, patients presenting with viral pneumonia were reported in Wuhan, China. A novel coronavirus was subsequently identified as the causative agent, and provisionally named 2019 novel coronavirus (2019-nCov or SARS-CoV-2) (Zhu N et al., N Engl J Med., 2020 Jan 24). The virus swiftly spread within and outside China, leading to the WHO declaring a Public Health Emergency of International Concern on January 30th, 2020. With the aim of rapid development of an assay for detection of the presence and/or absence of SARS-CoV-2 in a sample, and based on state of the art real-time RT-PCR technology, two targets, in the RNA-dependent RNA polymerase (RdRP or nsp12) and Single-stranded RNA-binding protein (nsp9) genes of the virus were identified herein by the inventors. Since mid-2020, emerging SARS-CoV-2 variants of concern due to their increased transmissibility and/or reduced sensitivity to neutralizing antibodies were reported in several countries and are currently spreading worldwide. To detect these new variants of SARS-CoV-2 specifically, two additional targets, in the nsp6 and N genes were identified herein by the inventors.

Coronaviruses are enveloped, positive single stranded RNA viruses. Coronaviruses have been identified in various mammalians hosts such as bats, camels, or mice, among others. Several coronaviruses are pathogenic to humans, leading to varying severity of symptoms (Cui et al., Nat Rev Microbiol. 2019 Mar;17(3):181-92). Highly pathogenic variants include the severe acute respiratory syndrome coronavirus (SARS-Cov) that emerged in China in 2002, resulting in -8000 human infections and 700+ deaths (Peiris et al., Nat Med., 2004 Dec;10(12 Suppl):S88-97) and the Middle East respiratory syndrome coronavirus (MERS-CoV), first detected in Saudi Arabia in 2012 and responsible for ~2500 human infections and 850+ deaths (Zaki et al., N Engl J Med., 2012 Nov 8;367(19):1814-20 ; Lee et al., BMC Infect Dis. 2017 Jul 14;17(1):498).

SARS-Cov-2 genome comprises the following open reading frames or ORFs, from its 5' end to its 3' end: ORF1a and ORF1b corresponding to the non-structural proteins forming the transcription-replication complex, and ORF-S, ORF-E, ORF-M and ORF-N corresponding to the four major structural proteins, spike surface glycoprotein (S), envelope protein (E), membrane glycoprotein (M) and nucleocapsid protein (N). It also comprises several accessory proteins like ORFs interspersed among or overlapping the structural genes and corresponding to proteins of unknown function.

SARS-Cov-2 RNA genome has a 5' methylated cap and a 3' polyadenylated tail, which allows the RNA to attach to the host cell's ribosome for translation. ORF1b encodes a protein called RNA-dependent RNA polymerase (RdRp or nsp12), which allows the viral genome to be transcribed into new RNA copies using the host cell's machinery. The RdRp is the first protein to be made; once the gene encoding the RdRp is translated, translation is stopped by a stop codon. RNA-dependent RNA polymerase (RdRp, RDR) is an enzyme that catalyzes the replication of RNA from an RNA template. This is in contrast to a typical DNA-dependent RNA polymerase, which catalyzes the transcription of RNA from a DNA template. RdRP is an essential protein encoded in the genomes of all RNA-containing viruses with no DNA stage. It catalyzes synthesis of the RNA strand complementary to a given RNA template. The RNA replication process is a two-step mechanism. First, the initiation step of RNA synthesis begins at or near the 3' end of the RNA template by means of a primer-independent (de novo), or a primer-dependent mechanism that utilizes a viral protein genome-linked (VPg) primer. The de novo initiation consists in the addition of a nucleoside triphosphate (NTP) to the 3'-OH of the first initiating NTP. During the following so-called elongation phase, this nucleotidyl transfer reaction is repeated with subsequent NTPs to generate the complementary RNA product. The protein nsp9 which is encoded by ORF1a may participate in viral replication by acting as Single-stranded RNA-binding protein. The protein nsp6, also encoded by ORF1a, plays a role in the initial induction of autophagosomes from host reticulum and later limits expansion of these phagosomes that are no longer able to deliver viral components to lysosomes.

In early 2020, the WHO published the various available protocols for SARS-CoV-2 detection by RT-PCR designed by national institutes of infectious diseases around the world: China CDC, China; Institut Pasteur, Paris, France; US CDC, USA; National Institute of Infectious Diseases, Japan; Charit6, Germany; HKU, Hong-Kong SAR; National Institute of Health, Thailand (Who Team: "Molecular assays to diagnose COVID-19: Summary table of available protocols).

Several variants of SARS-CoV-2 carrying mutations on the Spike protein with a predicted impact on the epidemiology of the Covid-19 disease emerged since mid-2020 and are currently spreading worldwide. These variants of concern were first reported in the UK (lineage B.1.1.7; notable mutations N501Y, 69-70del, P681H) and VOC-202102/02 (B.1.1.7 with E484K); South Africa (SA) (lineage B.1.351; notable mutations N501Y, E484K, K417N); Brazil (BR) (lineage P.1; notable mutations N501Y, E484K, K417T); UK and Nigeria (lineage B.1.525; notable mutations E484K, F888L, 69-70del) and are currently spreading to multiple countries around the world.

All these new variants of SARS-CoV-2 are characterized by an enhanced human-to-human transmissibility in comparison to earlier variants of the virus. The UK, SA and BR variants all share the mutation N501Y in the receptor-binding region (RBD), predicted to increase the spike's binding affinity towards the human ACE2 receptor. Variants SA and BR share an additional mutation in this region (K417T/N) suspected to contribute to further binding affinity to hACE2. The UK variant carries another mutation outside the RBD (del69/70) with a predicted impact on transmissibility.

Furthermore, the variants SA and BR share an additional mutation in the RBD (E484K) reported to enhance SARS-CoV-2 ability to escape the immune response (both natural and vaccine induced). Monoclonal and serum-derived antibodies are reported to be from 10 to 60 time less effective in neutralizing virus bearing the E484K mutation. The distinct mutation L452R carried by the Californian variant was shown to enhance SARS-CoV-2 immune evasion ability in previous studies. Some vaccines might see their efficacies reduced against these variants.

Consequently, these emerging variants of SARS-CoV-2 are of concern due to their increased transmissibility (UK, BR, SA). Furthermore, the reduced sensitivity to neutralizing antibodies of the variants carrying the mutation E484K (SA, BR) may compromise vaccine effectiveness.

There is an urgent need for new methods and reagents to identify with specificity and improved sensitivity the presence and/or absence of SARS-CoV-2 including variants thereof in samples. There is also a need to identify specifically the emerging variants of SARS-CoV-2, in particular UK, BR and SA, and to distinguish those that may compromise vaccine effectiveness, in particular BR and SA).

Specific mutations in the S gene are now reported to affect the efficacy of vaccines and neutralizing antibodies. In addition, convergent evolution seems to indicate that N501Y or E484K, or both, harboring strains are evolving and spreading rapidly with growing numbers of strongly associated and co-occuring mutations in different regions of SARS-CoV-2 genes. There is a concern that current tests will not only fail at detecting all Covid-19 positive patients, but also that specific detection of strains harboring mutations in the S gene will only be valid for a short period of time. Therefore, there is a need to provide PCR based test that are robust and stable over time in detecting SARS-CoV-2 and variants thereof, and provide in simplex or multiplex format, additional specific detections of variants harboring mutations in the S gene; such as the N501Y and/or E484K variants
The assay for detecting of the presence and/or absence of SARS-CoV-2 in a sample based on the analysis of RdRP (nsp12) and nsp9 genes of the virus as depicted hereinafter was designed and tested by the National Reference Center for Respiratory Virus, Institut Pasteur Paris, in panels of patients in France suspected to be infected by SARS-CoV-2 or in close contact with individuals known to be infected by SARS-CoV-2. Following the results, this PCR test showed 100% positive detection of about at least as low as 100 copies of target RNA per reaction in singleplex or 10 copies in multiplex using the selected 2 pairs of primers described below. This test is not reactive to other coronavirus, nor to other viruses causing respiratory infections, and distress in some patients. This test which is now used in routine by the National Reference Center for Respiratory Virus, Institut Pasteur Paris has been initially validated on a panel of SARS-CoV-2 of 600 positive and negative patients, including asymptomatic contact individuals, individuals returning from epidemic zone, and symptomatic patients. Within symptomatic patients, as there was also a concurrent epidemic of flu in France, negative patients with the SARS-CoV2 test of the invention were confirmed to be infected by flu or other respiratory diseases. The validation of this test allowed dispatch for diagnosis to reference hospitals in France and abroad, and within the international network of Institut Pasteur around the world.

### SUMMARY OF THE INVENTION

The inventors have designed a method comprising nucleic acid primers and probes and is based in part on the inventors' discovery that these nucleic acid primers and probes enable detection of the presence or absence of SARS-CoV-2 including variants thereof with very desirable sensitivity and specificity.

The inventors have identified targets, in the RNA-dependent RNA polymerase (RdRP or nsp12) and Single-stranded RNA-binding protein (nsp9) genes of the virus which are among the most conserved regions with less than 0.1% of mutations among all variants sequenced (more than 449,000 SARS-CoV-2 genomic sequences from GISAID database). Therefore, one of the main advantages of the method of the invention is that despite the emergence of SARS-CoV-2 variants, it still allows the detection of the presence of existing SARS-CoV-2 RNA variant in a sample. The fact that the target regions identified by the inventors are conserved and invariant would not be obvious at the priority date of the present application because the sequence analysis is a retrospective analysis performed on sequences, for which most part, were only available after the priority date of the present application. Furthermore, hotspot mutations were subsequently found in the RdRp gene (Pachetti et al., Journal of Translational Medicine, 2020, 18, 179; Published online: 22 April 2020).

The invention provides a multiplex method for specific detection of the presence or absence of a SARS-CoV-2 RNA in a sample as defined in claim 1, comprising:
subjecting a sample to two simultaneous amplification reactions:
(A) a first reverse transcription reaction with a first SARS-CoV-2-specific reverse primer to generate a first cDNA copy of SARS-CoV-2 RNA in the sample; amplifying any resultant first cDNA with the first reverse primer and a first SARS-CoV-2 specific forward primer; and detecting any first amplified product with a first SARS-CoV-2 specific probe; wherein the first SARS-CoV-2 specific reverse primer hybridizes to the sequence: 5'-ACAACACAACAAAGGGAG-3' (SEQ ID NO: 11) and the RNA equivalent thereof; the first SARS-CoV-2 specific forward primer hybridizes to the sequence: 5'-CAACAGGACTAAGCTCAT-3' (SEQ ID NO: 10) and the first SARS-CoV-2 specific probe hybridizes to the sequence: 5'-TACCGGCAGCACAAGACATCT-3' (SEQ ID NO: 12) or the complement thereof; and
(B) a second reverse transcription reaction with a second SARS-CoV-2-specific reverse primer to generate a second cDNA copy of SARS-CoV-2 RNA in the sample; amplifying any resultant second cDNA with the second reverse primer and a second SARS-CoV-2 specific forward primer; and detecting any second amplified product with a second SARS-CoV-2 specific probe; wherein the second SARS-CoV-2 specific reverse primer hybridizes to the sequence: 5'-CCTATATTAACCTTGACCAG-3' (SEQ ID NO: 14) and the RNA equivalent thereof, the second SARS-CoV-2 specific forward primer hybridizes to the sequence: 5'-CGAAATCATACCAGTTACC-3' (SEQ ID NO: 13), and the second SARS-CoV-2 specific probe hybridizes to the sequence: 5'-CCTGGCGTGGTTTGTATGA-3' (SEQ ID NO: 15) or the complement thereof; and
wherein said SARS-CoV-2 is chosen from SARS-CoV-2 variants of isolate Wuhan-Hu-1 carrying mutations in the Spike protein predicted to increase viral particles binding affinity towards the human ACE2 receptor and/or reduce viral particles sensitivity to neutralizing antibodies.

In some embodiments of the method, said variants carry one or more predicted mutations in the Spike protein selected from the group consisting of: E484K; K417N, N501Y, A570D, P681H, T716I, S982A, D1118H, del69/70 and del144/145.

In a more preferred embodiment of the method,
- the first SARS-CoV-2 specific reverse primer comprises the sequence 5'-CTCCCTTTGTTGTGTTGT-3' (SEQ ID NO: 2), and is no more than 21 bases in length; and
- the first SARS-CoV-2 specific forward primer comprises the sequence 5'-ATGAGCTTAGTCCTGTTG-3' (SEQ ID NO: 1), and is no more than 21 bases in length; and
- the first SARS-CoV-2 specific probe comprises the sequence 5'-AGATGTCTTGTGCTGCCGGTA-3' (SEQ ID NO: 3) or the complement thereof, and is no more than 24 bases in length.

In another preferred embodiment of the method,
- the second SARS-CoV-2 specific reverse primer comprises the sequence 5'- CTGGTCAAGGTTAATATAGG -3' (SEQ ID NO: 5), and is no more than23 bases in length; and
- the second SARS-CoV-2 specific forward primer consists of or comprises the sequence 5'- GGTAACTGGTATGATTTCG -3' (SEQ ID NO: 4), and is no more than 22 bases in length; and
- the second SARS-CoV-2 specific the probe consists of or comprises the sequence 5'- TCATACAAACCACGCCAGG -3' (SEQ ID NO: 6), or the complement thereof, and is no more than 22 bases in length.

In a preferred embodiment, the method is an RT-PCR method, preferably quantitative reverse-transcriptase PCR (QRT-PCR).

In a preferred embodiment of the method, the probe is labelled with 6-carboxy-fluorescein (6FAM) or hexachloro-6-carboxy-fluorescein (HEX) at the 5' end.

In a preferred embodiment of the method, the probe is labelled with black hole quencher 1 (BHQ1) at the 3'.

In a preferred embodiment, the sample is an environmental sample or a biological sample, preferably a body fluid sample, more preferably oral or respiratory tract secretions.

### DETAILED DESCRIPTION OF THE INVENTION

According to standard practice in the field of virology, the sequences of coronavirus genome (positive single stranded RNA) or fragments thereof (target sequences for SARS-CoV-2 RNA amplification) are disclosed in the DNA form. Therefore, the sequence SEQ ID NO: 19 is the DNA equivalent of SARS-CoV-2 RNA and the sequences SEQ ID NO: 16 and SEQ ID NO: 17 are the DNA equivalent of SARS-CoV-2 RNA target sequences for SARS-CoV-2 RNA amplification.

As used herein, SARS-Co-V2 refers to any isolate, strain, lineage or variant of SARS-CoV-2.

All the sequences are indicated in their 5' to 3' orientation.

As used herein, NC_045512 or NC_045512.2 refers to GenBank accession number NC_045512.2 accessed on July 18, 2020, corresponding to the complete genome sequence of SARS-CoV-2 isolate Wuhan-Hu-1. NC_045512.2 is used as SARS-CoV-2 genome reference sequence.

The SARS-CoV-2 isolate Wuhan-Hu-1 is also referred to as BetaCoV_Wuhan_WIV04_2019 (EPI_ISL_402124) or BetaCoV_Wuhan_IVDC-HB-05_2019 EPI_ISL_402121.

As used herein, positions X to Y of SARS-CoV-2 genomic sequence GenBank accession number NC_045512.2 (reference sequence), means that the indicated positions are determined by alignment with reference sequence or according to the numbering of the reference sequence. By simple alignment with reference sequence, one skilled in the art can easily determine the corresponding positions in the genomic sequence of another SARS-CoV-2 isolate, lineage, strain or variant.

As used herein, the positions in the SARS-CoV-2 Spike protein are indicated according to the numbering of SARS-CoV-2 Wuhan-Hu-1 isolate Spike protein sequence NCBI YP_009724390.1 as accessed on July 18, 2020.

As used herein, SARS-Co-V2 variant refers to an emerging SARS-Co-V2 variant of concern due to its increased transmissibility and/or enhanced ability to escape the immune response (natural and/or vaccine induced). In particular, the SARS-CoV-2 variant carries mutations in the Spike protein predicted to increase the spike's and/or virus particles binding affinity towards the human ACE2 receptor and/or reduce SARS-CoV-2 virus particles sensitivity to neutralizing antibodies. The neutralizing antibodies may be monoclonal antibodies or serum antibodies, in particular human serum antibodies. Examples of such mutations include in particular, E484K; K417T/N, N501Y, A570D, P681H, T716I, S982A, D1118H, del69/70 and del144/145. Examples of mutations which increase the spike's binding affinity towards the human ACE2 receptor include in particular, N501Y, K417T/N in the receptor binding domain (RBD) and 69-70del outside of the RBB. Examples of mutations which reduce SARS-CoV-2 virus sensitivity to neutralizing antibodies include in particular, E484K, present in the SA and BR variants. Preferred SARS-CoV-2 variants include the UK variant (lineage B.1.1.7; notable mutations N501Y, 69-70del, P681H; GISAID epi accession number B.1.1.7 - EPI_ISL_1001329); South Africa (SA) variant (lineage B.1.351; notable mutations N501Y, E484K, K417N; GISAID epi accession number B.1.351-EPI_ISL_1001390); Brazil (BR) variant (lineage P.1; notable mutations N501Y, E484K, K417T; GISAID epi accession number P.1 - EPI_ISL_1001385).

Based on the first sequences of SARS-CoV-2 made available on the GISAID database on January 11, 2020 (SEQ ID NO: 19), primers and probes (nCoV_IP2 and nCoV_IP4) were designed to target the nsp9 and RdRp genes spanning nt 12621-12727 and 14010-14116, respectively (positions according SARS-CoV, NC_004718). These positions correspond to nt 12669-12776 and 14059-14165 in SARS-CoV-2 sequence (SEQ ID NO: 19) or nt 12690 to 12797 and 14080 to 14186 in NC_045512.2.

The following table lists preferred embodiments of SARS-CoV-2 nucleic acid sequences of this disclosure, which were identified by the inventors as described in the examples. The primer/probe column lists the sequences of the forward primer, reverse primer, and probe used in the Example. The Target Sequence (reverse complement) column lists the reverse complement (i.e., target) of the primer/probe sequence. The sequences are represented as DNA but in alternative embodiments at least one of the nucleotides may be an RNA nucleotide.

**Table 1: Primers and probes specific for the nsp9 and RdRp gene target**

| ***nsp9 gene* / *nCoV*_*IP2*** | **Primer/Probe** | **Target Sequence (reverse complement)** |
|---|---|---|
| nCoV_IP2-12669Fw | ATGAGCTTAGTCCTGTTG (SEQ ID NO: 1) | CAACAGGACTAAGCTCAT (SEQ ID NO: 10) |
| nCoV_IP2-12759Rv | CTCCCTTTGTTGTGTTGT (SEQ ID NO: 2) | ACAACACAACAAAGGGAG (SEQ ID NO: 11) |
| nCoV_IP2-12696bProbe(+) | AGATGTCTTGTGCTGCCGG TA (SEQ ID NO: 3) | TACCGGCAGCACAAGACAT CT (SEQ ID NO: 12) |
| | | |

| ***RdRp gene* / *nCoV_IP4*** | | |
|---|---|---|
| nCoV_IP4-14059Fw | GGTAACTGGTATGATTTCG (SEQ ID NO: 4) | CGAAATCATACCAGTTACC (SEQ ID NO: 13) |
| nCoV_IP4-14146Rv | CTGGTCAAGGTTAATATAG G (SEQ ID NO: 5) | CCTATATTAACCTTGACCA G (SEQ ID NO: 14) |
| nCoV_IP4-14084Probe(+) | TCATACAAACCACGCCAGG (SEQ ID NO: 6) | CCTGGCGTGGTTTGTATGA (SEQ ID NO: 15) |

The invention is directed to a method for specific detection of SARS-CoV-2 as defined in the claims. The method can be used for the determination of whether or not SARS-CoV-2 is present in the sample.

The invention provides a multiplex method for detecting the presence or absence of a SARS-CoV-2 RNA in a sample, comprising: subjecting the sample to two simultaneous amplification reactions:
(A) a first reverse transcription reaction with a first SARS-CoV-2-specific reverse primer to generate a first specific cDNA copy of SARS-CoV-2 RNA in the sample, wherein the first reverse primer hybridizes to the sequence: 5'-ACAACACAACAAAGGGAG-3' (SEQ ID NO:11) and the RNA equivalent thereof; amplifying any resultant first cDNA with the first reverse primer and a first SARS-CoV-2 specific forward primer, wherein the first SARS-CoV-2-specific forward primer hybridizes to the sequence: 5'-CAACAGGACTAAGCTCAT-3' (SEQ ID NO: 10); and detecting any first amplified product with a first SARS-CoV-2-specific probe that hybridizes to the sequence: 5'-TACCGGCAGCACAAGACATCT-3' (SEQ ID NO: 12) or the complement thereof; and
(B) a second reverse transcription reaction with a second SARS-CoV-2-specific reverse primer to generate a second cDNA copy of SARS-CoV-2 RNA in the sample, wherein the second SARS-CoV-2-specific reverse primer hybridizes to the sequence: 5'-CCTATATTAACCTTGACCAG-3' (SEQ ID NO:14) and the RNA equivalent thereof; amplifying any resultant second cDNA with the second reverse primer and a second SARS-CoV-2-specific forward primer, wherein the second forward primer hybridizes to the sequence: 5'-CGAAATCATACCAGTTACC-3' (SEQ ID NO: 13); and detecting any second amplified product with a second SARS-CoV-2-specific probe that hybridizes to the sequence: 5'-CCTGGCGTGGTTTGTATGA-3' (SEQ ID NO: 15) or the complement thereof.

In a preferred embodiment of the method, the first reverse primer comprises the sequence 5'-CTCCCTTTGTTGTGTTGT-3' (SEQ ID NO: 2), and is no more than 21 bases in length; and/or the first forward primer comprises the sequence 5'-ATGAGCTTAGTCCTGTTG-3' (SEQ ID NO: 1), and is no more than 21 bases in length; and/or the probe comprises the sequence 5'-AGATGTCTTGTGCTGCCGGTA-3' (SEQ ID NO: 3) or the complement thereof, and is no more than 24 bases in length.

In a more preferred embodiment of the method, the first reverse primer consists of the sequence 5'-CTCCCTTTGTTGTGTTGT-3' (SEQ ID NO: 2); and/or the first forward primer consists of the sequence 5'-ATGAGCTTAGTCCTGTTG-3' (SEQ ID NO: 1); and/or the first probe consists of the sequence 5'-AGATGTCTTGTGCTGCCGGTA-3' (SEQ ID NO: 3), or the complement thereof.

In a still more preferred embodiment of the method, the first reverse primer consists of the sequence 5'-CTCCCTTTGTTGTGTTGT-3' (SEQ ID NO: 2); the first forward primer consists of the sequence 5'-ATGAGCTTAGTCCTGTTG-3' (SEQ ID NO: 1); and the first probe consists of the sequence 5'-AGATGTCTTGTGCTGCCGGTA-3' (SEQ ID NO: 3), or the complement thereof.

In a preferred embodiment of the method, the second reverse primer comprises the sequence 5'-CTGGTCAAGGTTAATATAGG-3' (SEQ ID NO: 5), and is no more than 23 bases in length; and/or the second forward primer comprises the sequence 5'-GGTAACTGGTATGATTTCG-3' (SEQ ID NO: 4), and is no more than 22 bases in length; and/or the probe comprises the sequence 5' TCATACAAACCACGCCAGG 3' (SEQ ID NO: 6), or the complement thereof, and is no more than 22 bases in length.

In a more preferred embodiment of the method, the second reverse primer consists of the sequence 5'-CTGGTCAAGGTTAATATAGG-3' (SEQ ID NO: 5); and/or the second forward primer consists of the sequence 5'-GGTAACTGGTATGATTTCG-3' (SEQ ID NO: 4); and/or the second probe consists of the sequence 5'-TCATACAAACCACGCCAGG-3' (SEQ ID NO: 6), or the complement thereof.

In a still more preferred embodiment of the method, the reverse primer consists of the sequence 5'-CTGGTCAAGGTTAATATAGG-3' (SEQ ID NO: 5); the forward primer consists of the sequence 5'-GGTAACTGGTATGATTTCG-3' (SEQ ID NO: 4); and the probe consists of the sequence 5'-TCATACAAACCACGCCAGG-3' (SEQ ID NO: 6), or the complement thereof.

In the various embodiments, the method of the disclosure may comprise a preliminary step of providing a sample, before subjecting the sample to the reverse transcription reaction.

In some embodiments, the sample is an environmental sample, such as air, soil, food, beverages, feed, water (e.g., fresh water, salt water, waste water, and drinking water), sewage, sludge, and surfaces or samples obtained from surface swipes. In preferred embodiments, the sample is a biological sample, for example, stool, tissue sample, and body fluid. Body fluid includes mucosal secretions, such as with no limitations oral and respiratory tract secretions (sputa, saliva and the like) blood, plasma, serum, urine, and cerebrospinal fluid. Samples include swabs such as oral or nasopharyngeal (NP) swabs, aspirate, wash or lavage. Samples for diagnostic tests for SARS-CoV-2 can be taken from the upper (nasopharyngeal/oropharyngeal swabs, nasal aspirate, nasal wash or saliva) or lower respiratory tract (sputum or tracheal aspirate or bronchoalveolar lavage (BAL). In some particular embodiments, the biological sample is a clinical sample from human individual suspected of having SARS-CoV-2, preferably a body fluid sample, more preferably oral or respiratory tract secretions.

The sample can be subjected to well-known isolation and purification protocols or used directly. For example, the sample can be subjected to a treatment to release/extract the nucleic acids of the sample and/or to remove proteins and other non-nucleic acid components of the sample using conventional techniques, such as those in the Examples.

Reverse transcription of the RNA of a coronavirus strain can be performed with a "reverse primer" specific for coronavirus. A "reverse primer" is one that, based on its 5'-3' orientation, can bind to a single-stranded RNA and serve to initiate generation of a complementary DNA (cDNA) copy of the RNA. The reverse transcription can be accomplished using well known and routine methods. The reaction mix for reverse transcription contains the reagents for the reaction, for example, a reverse primer, dNTPs (dATP, dCTP, dGTP and dTTP), a buffer, and a reverse transcriptase. Exemplary reaction conditions are set forth in the examples.

Amplification of the cDNA copy of a coronavirus strain generated by reverse transcription can be performed with a "forward primer" specific for coronavirus. A "forward primer" is one that, based on its 5'-3' orientation, can bind to a single-stranded antisense cDNA copy of an RNA generated by reverse transcription and serve to initiate generation of a double-stranded DNA copy of the RNA. The amplification can be accomplished using well known and routine methods. The reagent mix for amplification contains the reagents for the reaction, for example a forward primer, a reverse primer, dNTPs, a buffer, and a DNA polymerase.

In particular, the method of the disclosure is performed using a single RT-PCR reagent mix containing the reagents for the reverse transcription and amplification reactions. The reverse primer used for the reverse transcription reaction is also used for the amplification reaction.

In particular, the reverse transcription and amplification reactions are performed in a plastic or glass container, most preferably in the same container.

Amplification methods known in the art include RCA, MDA, NASBA, TMA, SDA, LCR, b-DNA, PCR (all forms including RT-PCR), RAM, LAMP, ICAN, SPIA, QB-replicase, or Invader. A preferred amplification method is the polymerase chain reaction (PCR) amplification. See, e.g., PCR Technology: Principles and Applications for DNA Amplification (Ed. H. A. Erlich, Freeman Press, NY, N.Y., 1992); PCR Protocols: A Guide to Methods and Applications (Eds. linis, et al., Academic Press, San Diego, Calif., 1990); Mattila et al., Nucleic Acids Res. 19, 4967 (1991); Eckert et al., PCR Methods and Applications 1, 17 (1991); PCR (Eds. McPherson et al., IRL Press, Oxford); and U.S. Pat. Nos. 4,683,202, 4,683,195, 4,800,159 4,965,188, and 5,333,675. More preferred PCR methods is real-time PCR, PCR-HRM (High-Resolution DNA Melting) (see Andriantsoanirina et al. Journal of Microbiological Methods, 78 : 165 (2009)) and PCR coupled to ligase detection reaction based on fluorescent microsphere (Luminex^{®} microspheres).

Amplification techniques include in particular isothermal methods and PCR-based techniques. Isothermal techniques include such methods as nucleic acid sequence-based amplification (NASBA), loop-mediated isothermal amplification (LAMP), helicase-dependent amplification (HDA), rolling circle amplification (RCA), and strand displacement amplification (SDA), exponential amplification reaction (EXPAR), isothermal and chimeric primer-initiated amplification of nucleic acids (ICANs), signal-mediated amplification of RNA technology (SMART) and others (see e.g. Asiello and Baeumner, Lab Chip; 11(8): 1420-1430, 2011).

In particular, the PCR technique quantitatively measures starting amounts of DNA, cDNA, or RNA. Examples of PCR-based techniques according to the invention include techniques such as, but not limited to, quantitative PCR (Q-PCR), reverse-transcriptase polymerase chain reaction (RT-PCR), quantitative reverse-transcriptase PCR (QRT-PCR), or digital PCR. These techniques are well known and easily available technologies for those skilled in the art.

In some embodiments, the method is an RT-PCR method, preferably quantitative reverse-transcriptase PCR (QRT-PCR).

Preferably, the method is a one-step real-time RT-PCR assay, for example, as described in the Examples.

A probe is used to detect the amplified product. The probe can be labeled with a fluorescent, radioactive, or enzymatic label. The amplified product can be detected with a specific detection chemistry such as fluorescence resonance energy transfer (FRET) probes, TAQMAN probes, molecular beacons, scorpion probes, fluorescently labeled (or other labeled) primers, lightup probes or a dye-based chemistry, DNA, PNA, LNA, or RNA including modified bases that bind to the amplified product to detect the sequence of interest.

Detection of the amplified products can be real-time (during the amplification process) or endpoint (after the amplification process). The method allows for detection of the amplification products in the same vessel as amplification occurs.

In particular, a DNA internal control is used to monitor the amplification reaction.

Specifically, a RNA internal control is used to monitor the reverse transcription and amplification reactions.

In particular, the method comprises reverse transcribing and amplifying an internal positive control. In particular, the internal positive control reverse primer comprises the sequence 5' ATATTGCAGCAGTACGCACACA-3' (SEQ ID NO: 8), and is from 19 to 25 bases in length; and/or the internal positive control forward primer comprises the sequence 5' ACAGGTACGTTAATAGTTAATAGCGT-3' (SEQ ID NO: 7), and is from 23 to 29 bases in length; and/or the internal positive control probe comprises the sequence 5' ACACTAGCCATCCTTACTGCGCTTCG 3' (SEQ ID NO: 9), or the complement thereof, and is from 23 to 29 bases in length.

In some embodiments of the method, the probe and the internal positive control probe are labelled with 6-carboxy-fluorescein (6FAM) or hexachloro-6-carboxy-fluorescein (HEX) at the 5' end.

In some embodiments of the method, the probe and the internal positive control probe are labelled with black hole quencher 1 (BHQ1) at the 3' end.

In some embodiments of the method, the coronavirus is SARS-CoV-2 and is not other conoravirus, nor other viruses causing respiratory diseases.

The primers of the disclosure are used for both reverse transcription of RNA and amplification of the resultant products. The primer sequences are selective to SARS-CoV-2 within the coronaviruses and also other prevalent viruses causing respiratory diseases. Furthermore, the primers of the disclosure target sequences which are among the most conserved regions with less than 0.1% of mutations among all variants sequenced (more than 449,000 SARS-CoV-2 genomic sequences from GISAID database). Therefore, one of the main advantages of the the primers of the disclosure is that despite the emergence of SARS-CoV-2 variants, they still allow the detection of the presence of existing SARS-CoV-2 RNA variant in a sample.

In particular, the primers are in a set of one forward primer and one reverse primer.

The "reverse primer" is an anti-sense primer, which is the primer for reverse transcription, and is conserved among coronavirus strains. The reverse primer is specific for SARS-CoV-2.

The first reverse primer hybridizes to the sequence: 5'-ACAACACAACAAAGGGAG-3' (SEQ ID NO: 11) and the RNA equivalent thereof and the second reverse primer hybridizes to the sequence: 5'-CCTATATTAACCTTGACCAG-3' (SEQ ID NO: 14) and the RNA equivalent thereof.

In a preferred embodiment, the first primer for reverse transcription comprises the sequence 5'-CTCCCTTTGTTGTGTTGT-3' (SEQ ID NO: 2), and is no more than 21 bases in length, or the second primer for reverse transcription comprises the sequence 5'- CTGGTCAAGGTTAATATAGG -3' (SEQ ID NO: 5), and is no more than 23 bases in length. Preferably, the first reverse primer consists of the sequence 5'-CTCCCTTTGTTGTGTTGT-3' (SEQ ID NO: 2) or the second reverse primer consists of the sequence 5'-CTGGTCAAGGTTAATATAGG-3' (SEQ ID NO: 5).

In this context, the term "hybridizes to" refers to the ability of the primer to form a double-stranded hybrid molecule with SARS-CoV-2 RNA (i.e., comprising the RNA equivalent of SEQ ID NO: 11 or 14) sufficient to produce a cDNA and to promote amplification of the cDNA under standard reverse transcription and amplification conditions such as those set forth in the example.

The "forward primer" is a sense primer, which is specific for a subset of SARS-CoV-2.

The first forward primer hybridizes to the sequence: 5'-CAACAGGACTAAGCTCAT-3' (SEQ ID NO: 10) and the second forward primer hybridizes to the sequence: 5'-CGAAATCATACCAGTTACC-3' (SEQ ID NO: 13).

In a preferred embodiment, the first forward primer comprises the sequence 5'-ATGAGCTTAGTCCTGTTG-3' (SEQ ID NO: 1), and is no more than 21 bases in length, or the second forward primer comprises the sequence 5'-GGTAACTGGTATGATTTCG -3' (SEQ ID NO: 4), and is no more than 22 bases in length. Preferably, the first forward primer consists of the sequence 5'-ATGAGCTTAGTCCTGTTG-3' (SEQ ID NO: 1) or the second forward primer consists of the sequence 5'-GGTAACTGGTATGATTTCG-3' (SEQ ID NO: 4).

In this context, the term "hybridizes to" refers to the ability of the primer to form a double-stranded hybrid molecule with SARS-CoV-2 RNA (i.e., comprising the RNA equivalent of SEQ ID NO: 10 or 13) sufficient to produce a cDNA and to promote amplification of the cDNA under standard reverse transcription and amplification conditions such as those set forth in the example.

The primer (reverse or forward) hybridizes to the target sequence or complement thereof and can be further extended in the presence of a nucleic acid polymerase to specifically amplify the target sequence. The primer sequence is substantially complementary to the target sequence or its complement. Substantially complementary means that the primer sequence is at least 80% identical, preferably at least 85%, 90%, 95% and 98% identical to the target sequence or its complement. The primer may comprise additional sequences (not complementary to the target sequence) at its 5' end. In particular, the primer comprises a sequence of at least 5, preferably 10 to 15 consecutive nucleotides which is 100% identical to the target sequence or its complement. More particularly, the primer sequence is 100% identical to the target sequence or its complement. Optionally, at least one primer of the pair includes a label (detectable moiety). In some more preferred embodiments, the reverse and forward primer is as described above.

The disclosure provides a first set of primers for use in the amplification of a SARS-CoV-2 RNA in a sample, wherein the primer set comprises: a first reverse primer that hybridizes to the sequence: 5'-ACAACACAACAAAGGGAG-3' (SEQ ID NO:11) and the RNA equivalent thereof; and a first forward primer that hybridizes to the sequence: 5'-CAACAGGACTAAGCTCAT-3' (SEQ ID NO: 10).

In particular, the primer set comprises: a first reverse primer comprising the sequence 5'-CTCCCTTTGTTGTGTTGT-3' (SEQ ID NO: 2), that is no more than 21 bases in length; and a first forward primer comprising the sequence 5'-ATGAGCTTAGTCCTGTTG-3' (SEQ ID NO: 1), that is no more than 21 bases in length.

Particularly, the first reverse primer consists of the sequence 5'-CTCCCTTTGTTGTGTTGT-3' (SEQ ID NO: 2); and the first forward primer consists of the sequence 5'-ATGAGCTTAGTCCTGTTG-3' (SEQ ID NO: 1).

The disclosure provides a second set of primers, wherein the primer set comprises: a second reverse primer that hybridizes to the sequence: 5'-CCTATATTAACCTTGACCAG-3' (SEQ ID NO: 14) and the RNA equivalent thereof; and a second forward primer that hybridizes to the sequence: 5'-CGAAATCATACCAGTTACC-3' (SEQ ID NO: 13).

In particular, the primer set comprises: a second reverse primer comprising the sequence 5' -CTGGTCAAGGTTAATATAGG -3' (SEQ ID NO: 5) that is no more than23 bases in length; or a second forward primer comprising the sequence 5'-GGTAACTGGTATGATTTCG-3' (SEQ ID NO: 4), that is no more than 22 bases in length.

Particularly, the second reverse primer consists of the sequence 5'-CTGGTCAAGGTTAATATAGG-3' (SEQ ID NO: 5); and the second forward primer consists of the sequence 5'-GGTAACTGGTATGATTTCG -3' (SEQ ID NO: 4).

The disclosure provides a set of primers for use in the amplification of a SARS-CoV-2 RNA in a sample, wherein the primer set comprises: a first reverse primer that hybridizes to the sequence: 5'-ACAACACAACAAAGGGAG-3' (SEQ ID NO:11) and the RNA equivalent thereof; a first forward primer that hybridizes to the sequence: 5'-CAACAGGACTAAGCTCAT-3' (SEQ ID NO:10); a second reverse primer that hybridizes to the sequence: 5'- CCTATATTAACCTTGACCAG-3' (SEQ ID NO:14) and the RNA equivalent thereof; and a second forward primer that hybridizes to the sequence: 5'-CGAAATCATACCAGTTACC-3' (SEQ ID NO:13).

In particular, the primer set comprises: a first reverse primer comprising the sequence 5'-CTCCCTTTGTTGTGTTGT-3' (SEQ ID NO: 2), that is no more than 21 bases in length; a first forward primer comprising the sequence 5'-ATGAGCTTAGTCCTGTTG-3' (SEQ ID NO: 1), that is no more than 21 bases in length; a second reverse primer comprising the sequence 5'-CTGGTCAAGGTTAATATAGG-3' (SEQ ID NO: 5), that is no more than 23 bases in length; and a second forward primer comprising the sequence 5'-GGTAACTGGTATGATTTCG-3' (SEQ ID NO: 4), that is no more than 22 bases in length.

Particularly, the first reverse primer consists of the sequence 5'-CTCCCTTTGTTGTGTTGT-3' (SEQ ID NO: 2); the first forward primer consists of the sequence 5'-ATGAGCTTAGTCCTGTTG-3' (SEQ ID NO: 1); the second reverse primer consists of the sequence 5'-CTGGTCAAGGTTAATATAGG -3' (SEQ ID NO: 5); and the second forward primer consists of the sequence 5'-GGTAACTGGTATGATTTCG-3' (SEQ ID NO: 4).

The probes of the disclosure are useful for detection of coronavirus nucleic acids. As referred to herein, the "probe" of the disclosure is linked to a detectable label suitable for use in the method the invention. The probe is specific for SARS-CoV-2 strains. The probe sequences are selective to SARS-CoV-2 within the coronaviruses and also other prevalent viruses causing respiratory diseases. Furthermore, just like the primers, the probes of the disclosure target sequences which are among the most conserved regions with less than 0.1% of mutations among all variants sequenced (more than 449,000 SARS-CoV-2 genomic sequences from GISAID database). Therefore, one of the main advantages of the the probes of the disclosure is that despite the emergence of SARS-CoV-2 variants, they still allow the detection of the presence of existing SARS-CoV-2 RNA variant in a sample.

A "detectable label" as used herein is a moiety, which can be detected directly or indirectly. In some embodiments, detection of the label involves directly detecting an emission of energy by the label (e.g., radioactivity, luminescence, optical). A label can also be detected indirectly by its ability to bind to or cleave another moiety, which itself may emit or absorb light of a particular wavelength (e.g., biotin, avidin, epitope tag such as the FLAG epitope, enzyme tag such as horseradish peroxidase). Particular detectable labels include radioactive labels, fluorescent labels, chemiluminescent labels, bioluminescent labels, and epitope tags. Particularly, the probe is labelled with the fluorescent dyes 6-carboxy-fluorescein (6FAM) or hexachloro-6-carboxy-fluorescein (HEX), more particularly at the 5 'end. More particularly, the probe is labelled at its 3' end with black hole quencher 1 (BHQ1).

Particularly, the probe is for use in the detection of amplification products of a SARS-CoV-2 RNA in a sample.

In particular, the first probe hybridizes to the sequence:
5'-TACCGGCAGCACAAGACATCT-3' (SEQ ID NO: 12) or the complement thereof, or the second probe hybridizes to the sequence 5'-CCTGGCGTGGTTTGTATGA-3' (SEQ ID NO: 15) or the complement thereof.

Particularly, the first probe consists of or comprises the sequence: 5'-AGATGTCTTGTGCTGCCGGTA-3' (SEQ ID NO: 3), or the complement thereof and is no more than 24 bases in length.

Particularly, the second probe consists of or comprises the sequence: 5'-TCATACAAACCACGCCAGG -3' (SEQ ID NO: 6), or the complement thereof and is no more than 22 bases in length.

More particularly, the first probe consists of the sequence 5'-AGATGTCTTGTGCTGCCGGTA-3' (SEQ ID NO: 3), or the complement thereof; or the second probe consists of the sequence 5'-TCATACAAACCACGCCAGG-3' (SEQ ID NO: 6), or the complement thereof.

In particular, the probe is labelled with 6-carboxy-fluorescein (6FAM) or hexachloro-6-carboxy-fluorescein (HEX) at the 5' end.

In particular, the probe is labelled with black hole quencher 1 (BHQ1) at the 3' end.

The method of the disclosure encompasses the inclusion of controls for the reverse transcription and/or amplification reactions. The DNA control of the invention is useful to monitor the amplification reaction.

In particular, the control is an internal positive control, for example, wherein the internal positive control reverse primer comprises the sequence 5'-ATATTGCAGCAGTACGCACACA-3' (SEQ ID NO: 8), and is from 19 to 25 bases in length; and/or wherein the internal positive control forward primer comprises the sequence 5'-ACAGGTACGTTAATAGTTAATAGCGT-3' (SEQ ID NO: 7), and is from 23 to 29 bases in length; and/or wherein the internal positive control probe comprises the sequence 5'-ACACTAGCCATCCTTACTGCGCTTCG-3' (SEQ ID NO: 9), or the complement thereof, and is from 23 to 29 bases in length.

In particular, a real-time RT-PCR assay includes in addition of unknown samples:
Two negative samples bracketing unknown samples during RNA extraction (negative extraction controls); and/or
Positive controls (in duplicate); when using in vitro synthesized transcripts as controls include five quantification positive controls (in duplicate) including 10⁵, 10⁴ and 10³ copies genome equivalent (ge) of in vitro synthesized RNA transcripts; and/or one negative amplification control.

The method according to the invention and as described above can be practiced on different samples; human and non-human animals, surfaces, soils, for diagnostic, epidemiology, surveillance, as well as to screen blood and tissue banks which may need to be tested against SARS-CoV2 as part of the screening for HIV1, HIV2, HBV, HCV, etc....

The practice of the present invention will employ, unless otherwise indicated, conventional techniques, which are within the skill of the art. Such techniques are explained fully in the literature.

The invention will now be exemplified with the following examples, which are not limitative, with reference to the attached drawings in which:

### FIGURES

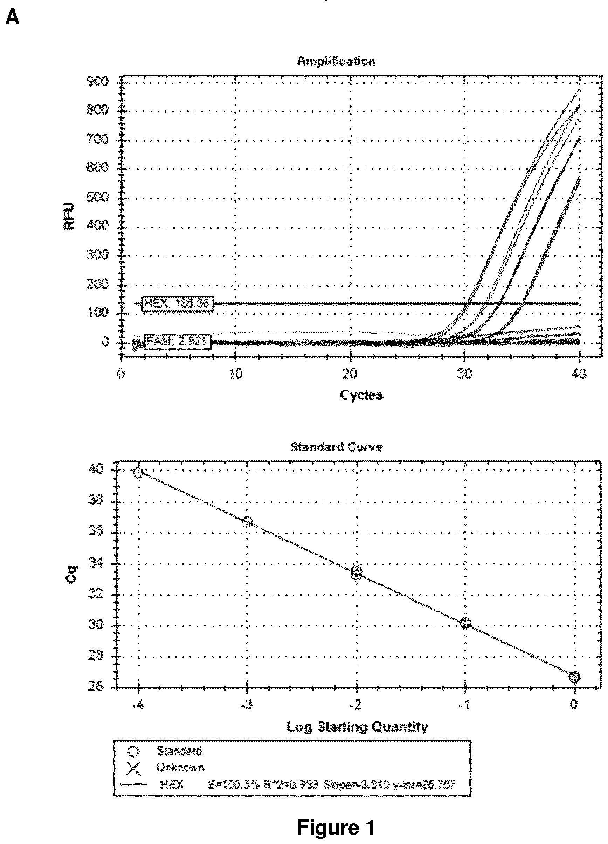

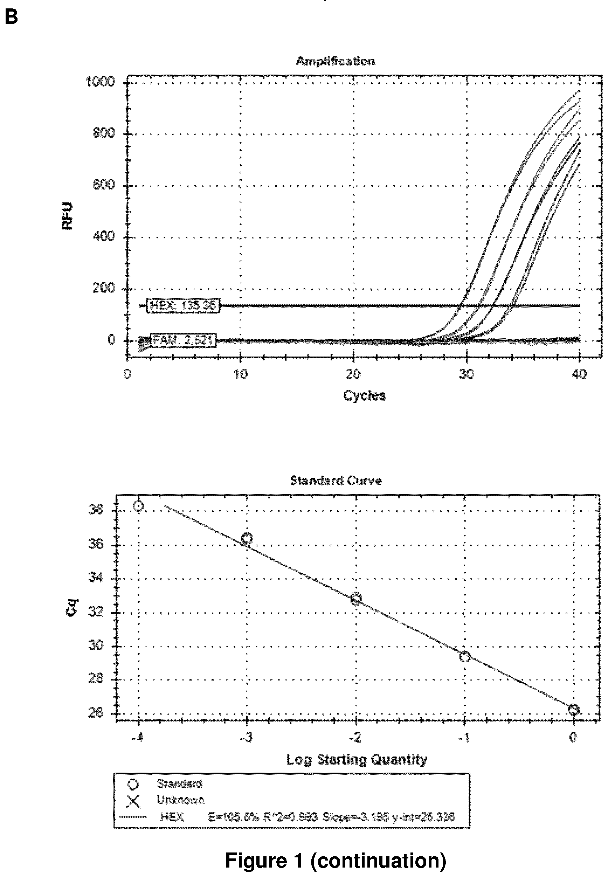

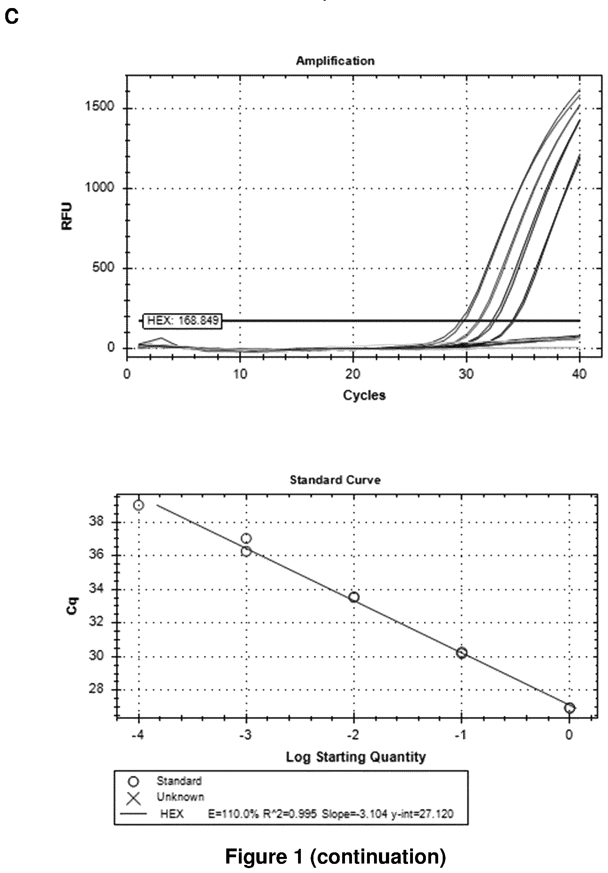

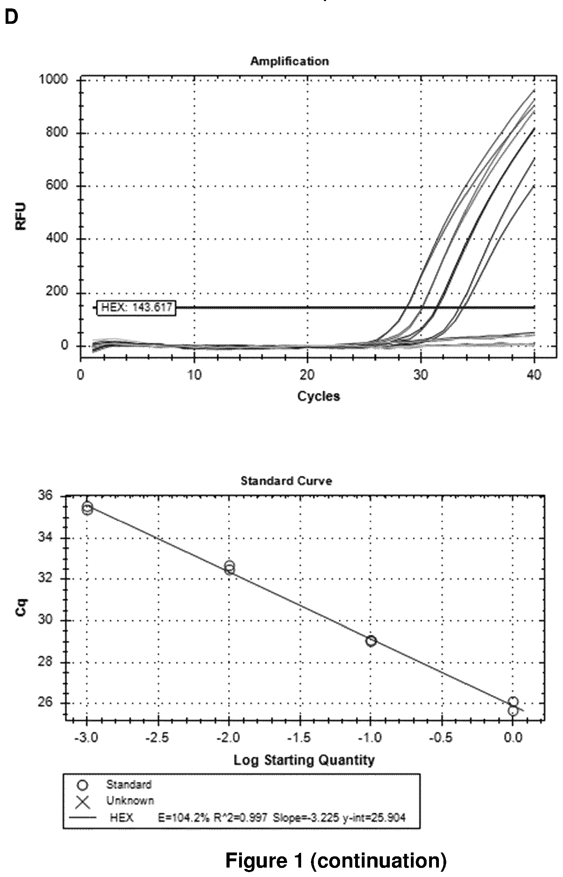

**Figure 1****:** RT-qPCR amplification plots of SARS-CoV-2 nsp6 gene in UK-SA-BR variants using different combination of sense and reverse primers. SARS-CoV-2 copy number was ranging from 4.0 10⁴ to 40 copies. The amplification efficiency (E) was determined by plotting of mean Cq values against five-fold copy number dilutions. The 4 primers-probe sets having an efficacy of nearly 100%: (A) MUT-As and MUT-Ar primers with MUT-probeA probe, (B) MUT-Bs and MUT-Br primers with MUT-probeA probe, (C) MUT-Cs and MUT-Cr primers with MUT-probeA probe and (D) MUT-Ds and MUT-Dr primers with MUT-probeA probe.

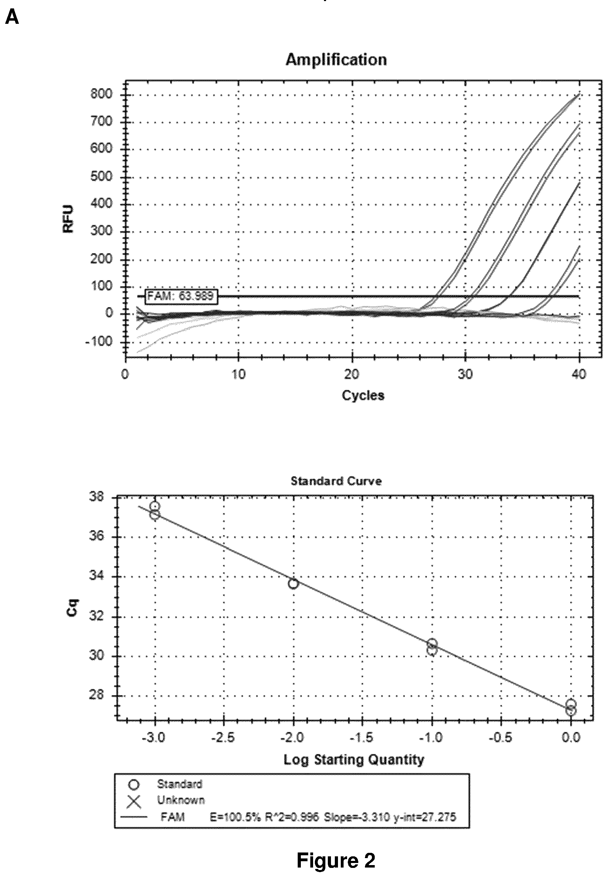

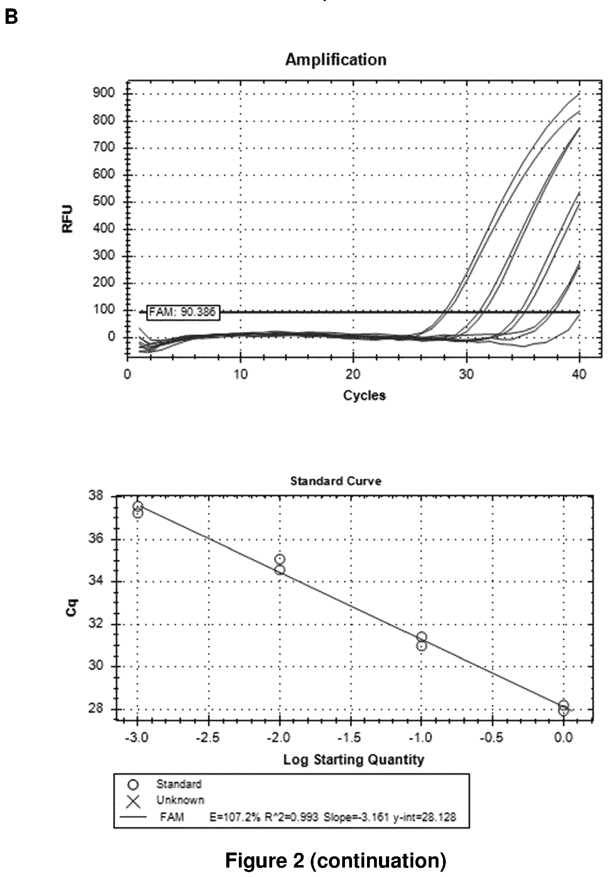

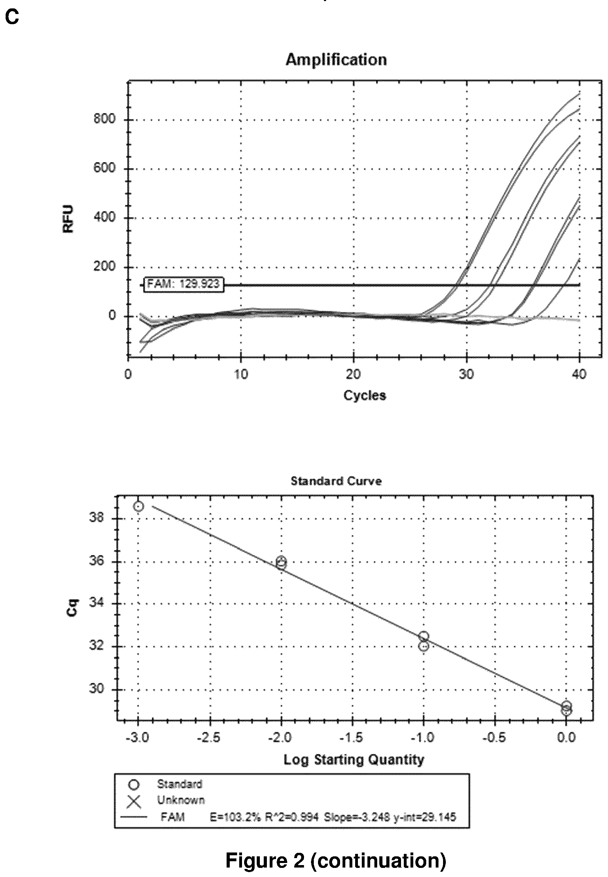

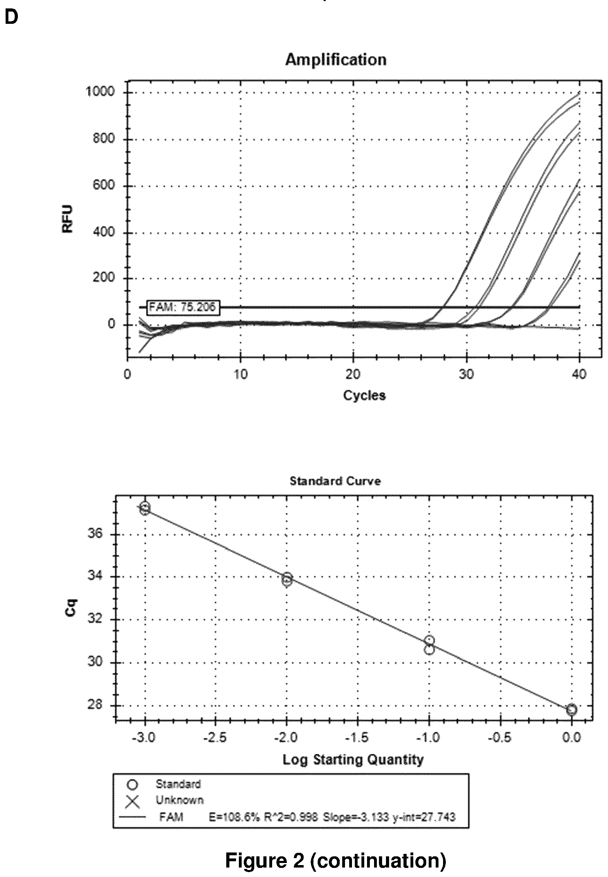

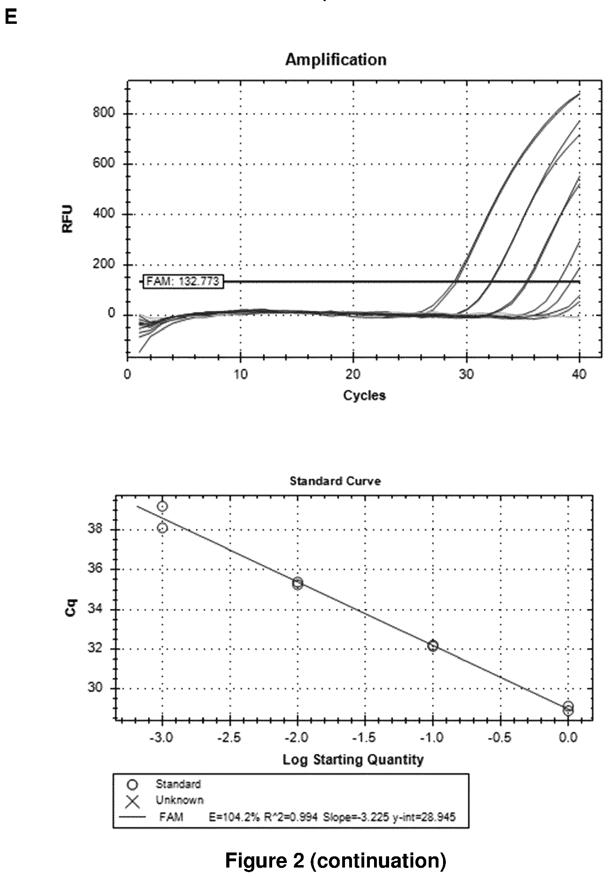

**Figure 2****:** RT-qPCR amplification plots of SARS-CoV-2 ORF8-N genes and intergenic region in UK variants using different combination of sense and reverse primers (A to E). A common probe was used for each reaction (UK-probe B). SARS-CoV-2 copy number in each reaction was ranging from 4.0 10⁵ to 40 copies. The amplification efficiency (E) was determined by plotting of mean Cq values against five-fold copy number dilutions.

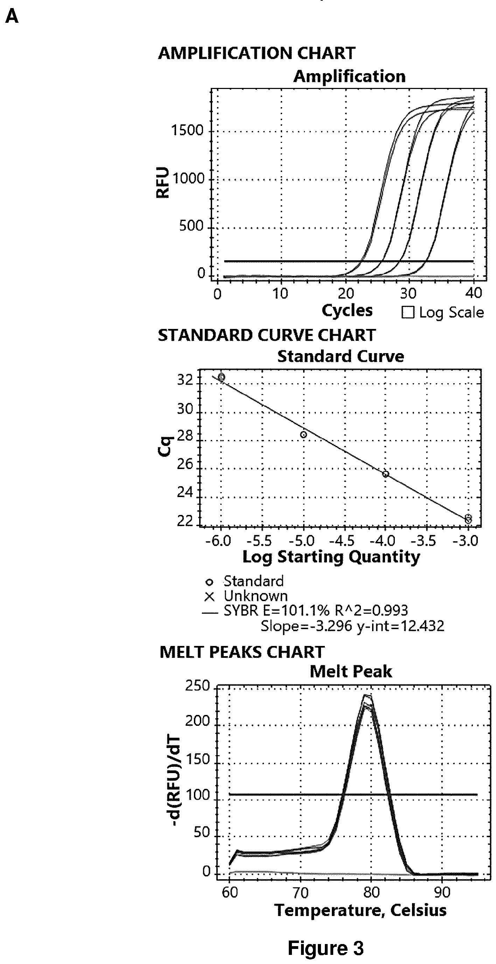

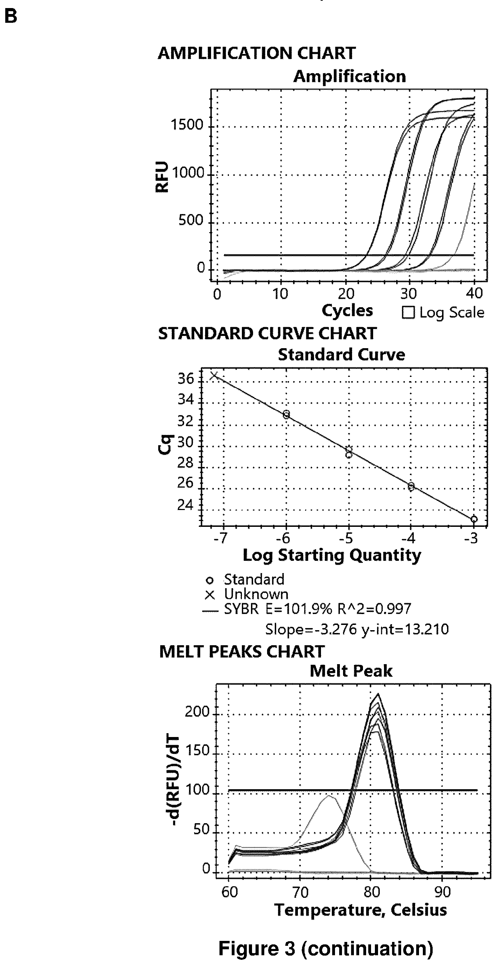

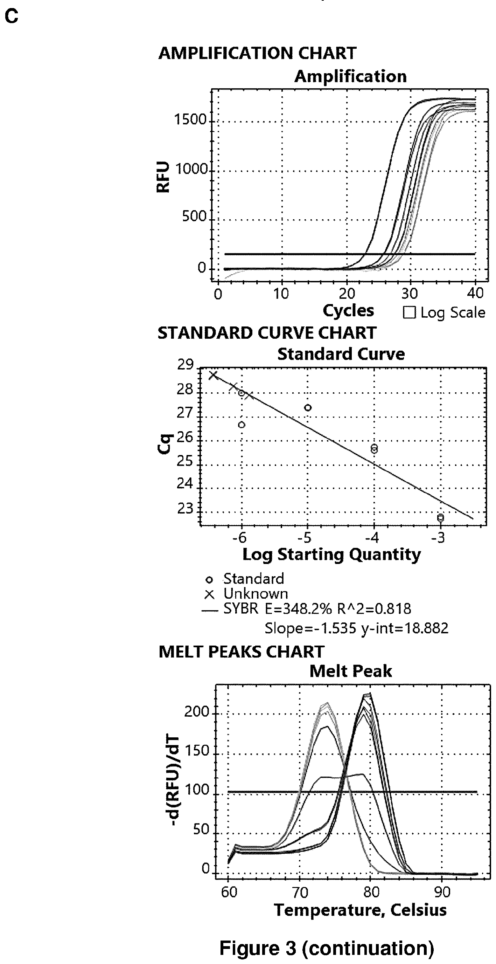

**Figure 3****:** Amplification and standard curves of UK-As+Ar (efficiency, E=101.1) and UK-Bs+Br (E=101.9). UK-Cs+Cr (bottom) is not specific (E=348.2 and see melting curve). The RNA used was CIBU-200132 (clinical sample, ancestral lineage) in a range of dilution from 1.0 E-03 to 1.0 E-06. Reaction were made with EVA green not with a specific probe, reason why we can use an ancestral SARS-CoV-2 RNA

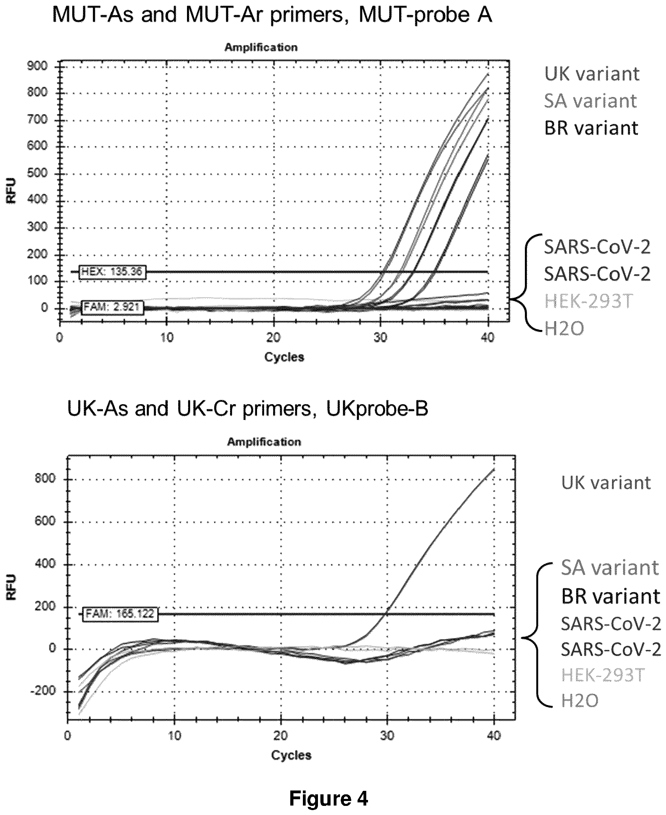

**Figure 4****:** Illustration of test specificities. UK-SAf-Br variants while MUT test (MUT: MUT-As+MUT-Ar+MUT-probe A) detects the 3 variants only. Likewise, UK-specific test (UK-as+UK-Cr+UKprobe-B) detect only UK variants not the ancestral clades or the SA-BR variants. The cDNA used are the same than previously (CIBU-200132, ancestral lineage detected in Greater Paris Hospitals in March 2020).

### EXAMPLES

### 1. Detection of SARS-CoV-2

This protocol describes procedures for the detection of SARS-CoV-2 for two targets in the nsp9 and RdRp genes (IP2 and !P4). Based on the first sequences of SARS-CoV-2 made available on the GISAID database on January 11, 2020 (SEQ ID NO: 19), primers and probes (nCoV_IP2 and nCoV_IP4) were designed to target the nsp9 gene spanning nt 12621-12727 and RdRp gene 14010-14116 (positions according SARS-CoV, NC_004718). These positions correspond to nt 12669-12776 and 14059-14165 in SARS-CoV-2 sequence (SEQ ID NO: 19) or nt 12690 to 12797 and 14080 to 14186 in NC_045512.2.

As a confirmatory assay, the E gene assay from the Charité protocol was used. (Corman VM, Landt O, Kaiser M, et al. Detection of 2019 novel coronavirus (2019-nCoV) by real-time RT-PCR. Euro Surveill 2020;25.)

### A. Kits

Kit Extraction NucleoSpin Dx Virus (Macherey Nagel 740895.50).
Superscript^{™} III Platinum^{®} One-Step Quantitative RT-PCR System. (Invitrogen 1732-020).

### B. Primers and Probes

**Tableau 2: Primer and probe sequences**

| **Name** | **Sequences (5'-3')** | **Length (bases)** | **PCR product size** | **Ref.** |
|---|---|---|---|---|
| ***nsp9 gene* / *nCo_IP2*** | | | | |
| nCoV_IP2-12669Fw | ATGAGCTTAGTCCTGTTG (SEQ ID NO: 1) | 18 | 108 bp | 1 |
| nCoV_IP2-12759Rv | CTCCCTTTGTTGTGTTGT (SEQ ID NO: 2) | 18 | | |
| nCoV_IP2-12696bProbe(+) | AGATGTCTTGTGCTGCCGGTA [5']Hex [3']BHQ-1 (SEQ ID NO: 3) | 21 | | |
| | | | | |

| ***RdRp gene* / *nCo V_IP4*** | | | | |
|---|---|---|---|---|
| nCoV_IP4-14059Fw | GGTAACTGGTATGATTTCG (SEQ ID NO: 4) | 19 | 107 bp | 1 |
| nCoV_IP4-14146Rv | CTGGTCAAGGTTAATATAGG (SEQ ID NO: 5) | 20 | | |
| nCoV_IP4-14084Probe(+) | TCATACAAACCACGCCAGG [5']Fam [3']BHQ-1 (SEQ ID NO: 6) | 19 | | |
| | | | | |

| ***E gene* / *E_Sarbeco (CoVE)*** | | | | |
|---|---|---|---|---|
| E_Sarbeco_F1 | ACAGGTACGTTAATAGTTAATAGCGT (SEQ ID NO: 7) | 26 | 113 bp | 2 |
| E_Sarbeco_R2 | ATATTGCAGCAGTACGCACACA (SEQ ID NO: 8) | 22 | | |
| E_Sarbeco_P1 | ACACTAGCCATCCTTACTGCGCTTCG [5']Fam [3']BHQ-1 (SEQ ID NO: 9) | 26 | | |

| | | | | |
|---|---|---|---|---|
| 1. National Reference Center for Respiratory Viruses, Institut Pasteur, Paris. 2. Corman VM, Landt O, Kaiser M, et al. Detection of 2019 novel coronavirus (2019-nCoV) by real-time RT-PCR. Euro Surveill 2020;25 | | | | |

Primer sets nCoV_IP2 and nCoV_IP4 can be multiplexed. Both reaction mixtures are described below.

PCR amplification regions (positions according to SARS-CoV, NC_004718):
nCoV_IP2: 12621-12727. The amplicon (108 bp) has the sequence SEQ ID NO: 16 which corresponds to positions 12669-12776 in SARS-CoV-2 sequence (SEQ ID NO: 19)
nCoV_IP4: 14010-14116. The amplicon (107 bp) has the sequence SEQ ID NO: 17 which corresponds to positions 14059-14165 in SARS-CoV-2 sequence (SEQ ID NO: 19)
E gene: 26141-26253. The amplicon (113 bp) has the sequence SEQ ID NO: 18 which corresponds to positions 26249-26361 in SARS-CoV-2 sequence (SEQ ID NO: 19).

### C. Nucleic Acid Extraction

RNA is extracted from specimens using the NucleoSpin Dx Virus (Macherey Nagel ref. 740895.50). RNA extracted from 100 µl of original sample, is eluted in 100 µl of elution buffer.

### D. Mix Preparation For All Separate Primer/Probe Combinations

All primers and probes described below were validated under the following conditions.

RT-PCR Mix kit: Invitrogen Superscript^{™} III Platinum^{®} One-Step qRT-PCR system (ref: 11732-088).

### Real-time PCR equipment: LightCycler 480 (96).

Adjustments may be required for the use of other kits or other real-time PCR instruments. All Assays used the same conditions. Primer and probe sequences, as well as optimized concentrations are shown in table below. A 25µl reaction was set up containing 5µl of RNA.

**Tableau 3: Simplex Mix**

| **Simplex Mix** | | **Vol (µl)** | **[final]** |
|---|---|---|---|
| | H2O PPI | 3.60 | |
| | Reaction mix 2X | 12.50 | 3 mM Mq |
| | MgSO4 (50mM) | 0.40 | 0.8 mM Mg |
| | Forward Primer (10µM) | 1.00 | 0.4 µM |
| | Reverse Primer (10µM) | 1.00 | 0.4 µM |
| | Probe (10µM) | 0.50 | 0.2 µM |
| | Superscriptlll RT/Platinum Taq Mix | 1.00 | |
| | **Final Volume** | **20.00** | |

**Tableau 4: Multiplex Mix**

| **Multiplex Mix (nCoV_IP2&IP4)** | | **Vol (µl)** | **final** |
|---|---|---|---|
| | H2O PPI | 1.3 | |
| | Reaction mix 2X | 12.50 | 3 mM Mg |
| | MqSO4 (50mM) | 0.40 | 0.8 mM Mg |
| | Forward Primer (10µM) | 1.00 | 0.4 µM |
| | Reverse Primer (10µM) | 1.00 | 0.4 µM |
| | Forward Primer (10µM) | 1.00 | 0.4 µM |
| | Reverse Primer (10µM) | 1.00 | 0.4 µM |
| | Probe (10µM) | 0.4 | 0.16 µM |
| | Probe (10µM) | 0.4 | 0.16 µM |
| | Superscriptlll RT/Platinum Taq Mix | 1.00 | |
| | **Final Volume** | **20.00** | |

### E. Controls

Each real-time RT-PCR assay includes in addition of unknown samples:
Two negative samples bracketing unknown samples during RNA extraction (negative extraction controls).

Positive controls (in duplicate); when using in vitro synthesized transcripts as controls include five quantification positive controls (in duplicate) including 10⁵, 10⁴ and 10³ copies genome equivalent (ge) of in vitro synthesized RNA transcripts.

One negative amplification control.

### F. Amplification cycles (Lightcycler System)

**Tableau 5: Amplification cycles**

| | | | | |
|---|---|---|---|---|
| Reverse transcription | 55°C | 20 min | x1 | |
| Denaturation | 95°C | 3 min | x1 | |
| Amplification | 95°C | 15 sec | x50 | Acquisition |
| | 58°C | 30 sec | | |
| Cooling | 40°C | 30 sec | x1 | |

### G. Sensitivity

### nCoV_IP and E_Sarbeco real-time RT-PCR

Sensitivity, in terms of 95% hit rate is about 100 copies of RNA genome equivalent per reaction (this amount of target sequences is always detected), the probability to detect lower amounts of virus decreases, but samples containing 10 copies could be detected with multiplex assay.

**Tableau 6: Sensitivity**

| | **Multiplex (Ct values)** | | **Simplex (Ct values)** |
|---|---|---|---|
| **RNA copies Of transcript** | **nCoV_I P2** | **nCoV_IP 4** | **E_Sarbeco** |
| 1,00E+07 | 21,67 | 21,97 | 24,72 |
| 1,00E+06 | 24,97 | 25,12 | 28,19 |
| 1,00E+05 | 28,00 | 27,88 | 30,96 |
| 1,00E+04 | 31,84 | 30,51 | 33,33 |

| | | | |
|---|---|---|---|
| Ct values may vary from instrument to instrument by up to 2 cycles, while the interval between two dilutions steps is constant (ΔCt). | | | |

### H. Specificity

Cross-reactivity with other respiratory viruses was tested with specimens known to be positive for a panel of respiratory viruses (influenza A(H1N1)pdm09, A(H3N2), B-Victoria, B-Yamagata; influenza C; RSV A, B; hBoV; hPIV; hMPV; HRV/enterovirus; adenovirus; hCoV (HKU1, OC43, 229E and NL63); MERS-CoV. None of the tested viruses showed reactivity with PCR2 and PCR4 using the IP2 and IP4 sets of primers and probes described in the Table above.

### I. Positive Control For SARS-CoV-2 Real Time RT-PCR

Positive control for real-time RT-PCR is an in vitro transcribed RNA derived from strain BetaCoV_Wuhan_WIV04_2019 (EPI_ISL_402124). The transcript contains the amplification regions of the RdRp and E gene as positive strand. Each microtube contains 1011 copies of target sequences diluted in yeast tRNA, and lyophilised.

### Reconstitution of transcribed RNA

Add 100 µl of RNase/DNAse-free H2O to obtain a solution at a concentration of 109 copies/µl. Store at -80°C. Dilute to prepare a master bank at 2x106 copies/µl. Store at -80°C.

From this prepare a working bank of reagent at 2x104 copies/µl in order to avoid freeze/thaw cycles. Working tubes may be stored at -20°C for less than one week.

This test which is now validated on a panel of SARS-CoV-2 of 600 positive and negative patients, including asymptomatic contact individuals, individuals returning from epidemic zone, and symptomatic patients. Within symptomatic patients, as there is also a concurrent epidemic of flu in France, negative patients with the SARS-CoV2 test of the invention were confirmed to be infected by flu or other respiratory diseases. The validation of this test will now allow dispatch for diagnosis to reference hospitals in France and abroad, and within the international network of Institut Pasteur around the world.

### J. Conservation of the region amplified by primer sets nCoV_IP2 and nCoV_IP4

More than 433,000 SARS-CoV-2 genomic sequences from GISAID database retrieved on February 6^{th}, 2021 were aligned to assess the degree of conservation of the gene sequences targeted by primer sets nCoV_IP2 and nCoV_IP4.

The multiple sequence alignment comprised 26% of genomic sequences from UK variant, 0.5% of genomic sequences from SA variant and 0.1% of genomic sequences from BR variant.

**Tableau 7: Conservtion of nsp9 and RdRp gene targets**

| Genes | Start (NC_045512) | End (NC_045512) | region size | conservation (in 433 425 compl genomes) | Primer set |
|---|---|---|---|---|---|
| nsp8-nsp9 | 12401 | 12865 | 465 | 0,999007799 | nCoV _IP2 |
| NIRAN-RORP-nsp12 | 13764 | 14340 | 577 | 0,999188415 | nCoV_IP4 |

The sequences of SARS-CoV-2 spanning nt 12401-12865 and 13764 - 14340 (positions according SARS-CoV isolate Wuhan-Hu-1, NC_045512) are among the most conserved regions with less than 0.1% of mutations among all variants sequenced. These regions comprise the sequences targeted by primer sets nCoV_IP2 and nCoV_IP4 respectively.

As an example, the less conserved region in the RdRp gene is a 101nt sequence spanning nt 14362-14462 with of conservation of 0.9933455.

### K. Conservation of SARS-CoV-2 regions hybridized by primer and probe sets nCoV_IP2 and nCoV_IP4

More than 450,000 SARS-CoV-2 genomic sequences from GISAID database retrieved on February 1^{st}, 2021, were aligned to assess the degree of conservation of each nucleotide of the regions hybridized by primer and probe sets nCoV_IP2 and nCoV_IP4.

Among the 451,405 sequences available, genomic sequences derived from non-human host samples, unknown host samples, environmental samples, cell cultures, samples with a date posterior to February 1^{st}, 2021, duplicate samples (same individual, same sample), as well as sequences inferior to 10nt or longer than 40,000nt, and sequences with non-standard letter code were removed prior alignment (-1,000 sequences).Repartition of the aligned sequences among the clades were as follow:

**Tableau 8: Repartition of the aligned sequences among the clades**

| Nb occurrences | % | Clade |
|---|---|---|
| 16914 | 3,75% | 19A |
| 9079 | 2,01% | 19B |
| 87153 | 19,31% | 20A |
| 11933 | 2,64% | 20A.(EU2) |
| 105420 | 23,36% | 20B |
| 48354 | 10,72% | 20C |
| 5092 | 1,13% | 20D |
| 99046 | 21,95% | 20E (EU1) |
| 12605 | 2,79% | 20F |
| 16207 | 3,59% | 20G |
| 774 | 0,17% | 20H/501Y.V2 (SA variant) |
| 38590 | 8,55% | 20I/501Y.V1 (UK variant) |
| 59 | 0,01% | 20J1501Y.V3 (BR variant) |

The degree of conservation of each nucleotide of the regions hybridized by primers and probe sets nCoV_IP2 and nCoV_IP4 and adjacent regions are presented in **Tables 27 to 32** (one per primer and probe).

Nucleotides in regions targeted by primers and probe sets nCoV_IP2 and nCoV_IP4 are conserved from at least 99,5 % up to 99,9 %.

Despite the emergence of SARS-CoV-2 variants, primers and probe sets nCoV_IP2 and nCoV_IP4 still allow the detection of the presence of existing SARS-CoV-2 RNA variant in a sample even if they were designed based on the first sequences of SARS-CoV-2 made available on the GISAID database on January 11, 2020.

### 2. Detection of UK, SA and BR SARS-CoV-2 variants

More than 433,000 SARS-CoV-2 genomic sequences from GISAID database retrieved on February 6^{th}, 2021 were aligned to identify a sequence feature specific of the Brazil (BR) variant (also known as P.1), the United-Kingdom (UK) or Kent variant (also known as B.1.1.7) and the South Africa (SA) variant (also known as B.1.351).

The multiple sequence alignment comprised 111,416 genomic sequences of UK variant, 2423 of the genomic sequences of SA variant and 429 genomic sequences of BR variant.

A 9 nucleotides deletion in ORF1a nsp6 gene spanning nt 11288-11296 (positions according SARS-CoV isolate Wuhan-Hu-1, NC_045512) is present only in BR, UK and SA variants.

This protocol describes procedures for the detection this 9 nucleotides deletion.

Based on the sequences of SARS-CoV-2 made available on the GISAID database by National Reference Center for Respiratory Viruses, Institut Pasteur, Paris (Table 9), primers and probes were designed to target the deletion in nsp6 gene spanning nt 11288-11296 (positions according SARS-CoV isolate Wuhan-Hu-1, NC_045512).

**Table 9 : GISAID accession numbers of UK, SA and BR genomes used as references for the present study and number of variant genomes used in the alignment to identify a sequence feature specific of these all 3 variants**

| **Variant** | **GISAID epi accession number** | **Nb of aligned sequences** |
|---|---|---|
| UK | B.1.1.7-EPI_ISL_1001329 | 11416 |
| SA | B.1.351- EPI_ISL_1001390 | 2423 |
| BR | P.1 - EPI_ISL_1001385 | 429 |

### A. Kits

iScript^{™} Advanced cDNA Synthesis Kit for RT-qPCR (Bio-Rad) for the reverse transcription step.

iTaq^{™} Universal SYBR^{®} Green Supermix (Bio-Rad) or
EurobioGreen qPCR Mix (Eurobio) for the DNA amplification and quantification step.

### B. Primers and probes

Primers were designed to allow the amplification of all SARS-CoV-2 viruses whereas the probes were designed to detect specifically the deletion in nsp6 gene spnning nt 11288-11296 (positions according to SARS-CoV-2 isolate Wuhan-Hu-1 NC_045512).

**Table 10: Primer and probe sequences**

| Name | Sequence (5' - 3') | Orientation on the genome | Position NC_04551 2 | Position NC_04551 2 | SE Q ID NO |
|---|---|---|---|---|---|
| MUT-As | GCTAGTTGGGTGATGCGTATT | FW | 11234 | 11254 | 20 |
| MUT-Bs | TGGTCTATATGCCTGCTAGTTG | FW | 11220 | 11241 | 21 |
| MUT-Cs | GATGCGTATTATGACATGGTTGG | FW | 11245 | 11267 | 22 |
| MUT-Ds | TCTCTTGCCACTGTAGCTTATT | FW | 11192 | 11213 | 23 |
| MUT-Fs | GTGATGCGTATTATGACATGGTT | FW | 11243 | 11265 | 24 |
| MUT-Ar | CTCCTAGCACCATCATCATACAC | RV | 11363 | 11385 | 25 |
| MUT-Br | GCTGATGCATACATAACACAGTC | RV | 11306 | 11328 | 26 |
| MUT-Cr | CAGTTCTTGCTGTCATAAGGATTAG | RV | 11339 | 11363 | 27 |
| MUT-Dr | GATTAGTAACACTACAGCTGATGC | RV | 11321 | 11344 | 28 |
| MUT-probeA | TGGTTGATACTAGTTTGAAGCT | FW | 11271 | 11301 | 29 |
| MUT-probeB | GCTTCAAACTAGTATCAACCATATC C | RV | 11266 | 11297 | 30 |

### C. Nucleic Acid Extraction and SARS-CoV-2 variant extracts

RNA is extracted from specimens using the NucleoSpin Dx Virus (Macherey Nagel ref. 740895.50). RNA extracted from 100 µl of original sample, is eluted in 100 µl of elution buffer.

**Table 11: SARS-CoV-2 variant strains used to test primers and probes**

| **Origin** | **Variant** | **Clades** | **Pango lineages** | |
|---|---|---|---|---|
| France | CIBU-200132 | | | CIBU, Institut Pasteur, Paris |
| France | D614G | G | | NRC for Respiratory Viruses, Institut Pasteur, Paris |
| UK | 201/501Y.V1 | GR | B.1.1.7 | NRC for Respiratory Viruses, Institut Pasteur, Paris |
| SA | 20H/501Y.V2 | GH | B.1.351 | NRC for Respiratory Viruses, Institut Pasteur, Paris |
| BR | 20J/501Y P1 | GR | P.1 | NRC for Respiratory Viruses, Institut Pasteur, Paris |

### D. Mix Preparation

All primers and probes described below were validated under the following conditions.

A variable volume of RNA was reverse transcribed using iScript Advanced cDNA synthesis kit for RT-qPCR (Bio-Rad) with 4µl of 5x iScript Advanced Reaction Mix and 1µl of iScript Advanced Reverse Transcriptase in a final volume of 20µl for 30 min 42°C, 5min 85°C in T100 Thermal Cycler (Bio-Rad)

Adjustments may be required for the use of other kits or other real-time PCR instruments.

All Assays used the same conditions. Primer and probe sequences, as well as optimized concentrations are shown in table below. A 20µl reaction was set up containing 2µl of RNA, 400nM of primers, and optionally 200nM of probe.

**Tableau 12: Simplex Mix**

| **Simplex Mix with SYBRGreen^{®} dye** | | **Vol (µl)** | **[final]** |
|---|---|---|---|
| | H₂O PPI | 6.4 | |
| | SYBR qreen mix 2X | 10 | 1X |
| | Forward Primer (10µM) | 0.8 | 0.4 µM |
| | Reverse Primer (10µM) | 0.8 | 0.4 µM |
| | **Final Volume** | **20.00** | |

**Tableau 13: Multiplex Mix**

| **Simplex Mix with a probe** | | **Vol (µl)** | **[final]** |
|---|---|---|---|
| | H₂O PPI | 6 | |
| | SYBR qreen mix 2X | 10 | 1X |
| | Forward Primer (10µM) | 0.8 | 0.4 µM |
| | Reverse Primer (10µM) | 0.8 | 0.4 µM |
| | Probe (10µM) | 0.4 | 0.2 µM |
| | **Final Volume** | **20.00** | |

### E. Controls

All primers and probes were tested by RT-qPCR with an RNA extract form HEK-293T (Human Embryonic Kidney) human cell line or without RNA template (H₂O).

### F. Amplification cycles

**Tableau 14: Amplification cycles**

| | | | | |
|---|---|---|---|---|
| Denaturation | 95°C | 2 min | x1 | |
| Amplification | 95°C | 5 sec | X40 | Acquisition |
| | 60°C | 30 sec | | |
| Cooling | 40°C | 30 sec | x1 | |

### G. Analytical specificity of the primer pairs

Since the designed probes target the 9 nucleotides deletion, it was possible to select the best primer pairs on ancestral SARS-CoV-2 RNA (extracted from clinical sample CIBU-200132 strains) with a DNA intercalant dye before testing combinations of the best primer pairs with a probe.

The specificity of the primer pairs was assessed using serial 10-fold dilutions of the genomic SARS-CoV-2 and by analyzing the melt curve.

### H. Amplification efficiencies

Based on the best primer pairs, the following combinations were tested:
- MUT-As, MUT-Ar, MUT-probeA
- MUT-Bs, MUT-Br, MUT-probeA
- MUT-Bs, MUT-Br, MUT-probeB
- MUT-Cs, MUT-Cr, MUT-probeA
- MUT-Ds, MUT-Dr, MUT-probeA
- MUT-Ds, MUT-Dr, MUT-probeB
- MUT-Fs, Mut-Cr, no probe.

All combinations were tested on RNA extract from UK, SA or BR variant, ancestral SARS-CoV-2, and controls (HEK-293T and H₂O) (Figure 1 and 4).

The following quantification cycle (Cq) values were obtained

**Table 15: Cq values obtained with each primers-probe combination for RNA samples from UK, SA and BR variants vs ancestral SARS-CoV-2. (nd: not detected)**

| | | **Cq MUT-As MUT - Ar MUT-probeA** | **Cq MUT-Bs MUT-Br MUT-probeA** | **Cq MUT-Cs MUT-Cr MUT-probeA** | **Cq MUT-Ds MUT-Dr MUT-probeA** |
|---|---|---|---|---|---|
| **Origin** | **Test (copies)** | | | | |
| France (ancestral) | 3.0 10⁵ | nd | nd | nd | nd |
| UK | 1.0 10⁵ | 30.165 | 29.355 | 29.15 | 28.475 |
| SA | 7.5 10⁴ | 31.725 | 30.965 | 30.575 | 29.89 |
| BR | no titration available | 34.9 | 33.86 | 33.6 | 33.33 |
| BR | no titration available | 32.92 | 32.225 | 31.925 | 31.195 |

No amplification was detected in controls.

The amplification efficiency (E) was determined by plotting the mean Cq values against five different copy numbers (10-fold dilutions).

**Table 16 : Cq values obtained with each primers-probe combination for a determined copy number of UK RNA, the dilution range being used as the standard curve.**

| **UK RNA range (Copy nb)** | Cq | Cq | Cq | Cq |
|---|---|---|---|---|
| | MUT-A primers | MUT-B primers | MUT-C primers | MUT-D primers |
| | MUT-probe-A | MUT-probe-A | MUT-probe-A | MUT-probe-A |
| 4.10⁵ | 26.49 | 25.98 | 26.98 | 26.36 |
| 4.10⁴ | 30.02 | 29.14 | 30.25 | 29.45 |
| 4.10³ | 33.27 | 32.50 | 33.55 | 33.06 |
| 4.10² | 36.53 | 36.12 | 36.66 | 35.99 |
| 40 | 39.71 | 38.05 | 39.02 | N/A |

**Table 17 : Efficiency of the primers sets to detect simultaneously the UK, SA and BR variants.**

| **Primers set (with MUT-probeA probe)** | **Efficiency (%)** |
|---|---|
| **MUT-A** | 100.5 |
| **MUT-B** | 105.6 |
| **MUT-C** | 110.0 |
| **MUT-D** | 104.2 |

Among the tested combinations, only the 4 following sets have an amplification efficiency of at least 100%:
- MUT-As, MUT-Ar, MUT-probeA
- MUT-Bs, MUT-Br, MUT-probeA
- MUT-Cs, MUT-Cr, MUT-probeA
- MUT-Ds, MUT-Dr, MUT-probeA

Since these 4 primers and probe sets does not detect ancestral SARS-CoV-2 virus or controls, they enable the specific detection of the UK, SA and BR variants.

### 3. Detection of UK SARS-CoV-2 variant

More than 433,000 SARS-CoV-2 genomic sequences from GISAID database retrieved on February 6^{th}, 2021 were aligned to identify a sequence feature specific of the United-Kingdom (UK) or Kent variant (also known as B.1.1.7).

The multiple sequence alignment comprised 111,416 genomic sequences of UK variant, 2423 of the genomic sequences of SA variant and 429 genomic sequences of BR variant.

Mutations of 3 consecutive nucleotides (GAT into CTA) spanning nt 28280-28282 (positions according SARS-CoV isolate Wuhan-Hu-1, NC_045512) corresponding to the 3^{rd} codon of the Nucleocapsid (N) gene, and leading to a mutation of the 3^{rd} amino acid (ASP into LEU) in protein N, is present only in the UK variant.

This protocol describes procedures for the detection this codon mutation.

Based on the genomic sequences of SARS-CoV-2 variants made available on the GISAID database by National Reference Center for Respiratory Viruses, Institut Pasteur, Paris (Table 18) and the genomic sequence of SARS-CoV isolate Wuhan-Hu-1 (NC_045512), primers and probes were designed to target the codon mutation in N gene spanning nt 28280-28282 (positions according SARS-CoV isolate Wuhan-Hu-1, NC_045512).

**Table 18: GISAID accession numbers of UK, SA and BR genomes used as references for the design of probes and primer and number of variant genomes used in the multiple sequence alignment to identify a sequence feature specific of the UK variant.**

| **Variant** | **GISAID epi accession number** | **Nb of aligned sequences** |
|---|---|---|
| UK | B.1.1.7 - EPI_ISL_1001329 | 111416 |
| SA | B.1.351- EPI_ISL_1001390 | 2423 |
| BR | P.1 - EPI_ISL_1001385 | 429 |

### A. Kits

iScript^{™} Advanced cDNA Synthesis Kit for RT-qPCR (Bio-Rad) for the reverse transcription step.

iTaq^{™} Universal SYBR^{®} Green Supermix (Bio-Rad) or EurobioGreen qPCR Mix (Eurobio) for the DNA amplification and quantification step.

### B. Primers and Probes

Primers were designed to allow the amplification of all SARS-CoV-2 viruses whereas the probes were designed to detect specifically the codon mutation in N gene spanning nt 28280-28282 (positions according SARS-CoV isolate Wuhan-Hu-1, NC_045512).

Primers and probe specificity for all variants or UK variant respectively were verified *in silico* by BLAST analysis on the genomic sequences retrieved from the GISAID database.

**Tableau 19: Primer and probe sequences**

| Name | Sequence (5' - 3') | Orien tation | Position NC_045 512 | Position NC_045 512 | SEQ ID NO |
|---|---|---|---|---|---|
| UK-As | AGTGCGTTGTTCGTTCTATGA | FW | 28190 | 28210 | 31 |
| UK-Bs | AGTATCATGACGTTCGTGTTGT | FW | 28222 | 28243 | 32 |
| UK-Cs | CGTTGTTCGTTCTATGAAGACTTT | FW | 28194 | 28217 | 33 |
| UK-Ds | TCATGACGTTCGTGTTGTTTT | FW | 28226 | 28246 | 34 |
| UK-D2s | TGCGTTGTTCGTTCTATGAAG | FW | 28192 | 28212 | 35 |
| UK-Es | TTCTCCATTCTGGTTACTGC | RV | 28347 | 28366 | 36 |
| UK-Fs | TGACGTTCGTGTTGTTTTAG | RV | 28229 | 28248 | 37 |
| UK-Ar | AGGGTCCACCAAACGTAATG | RV | 28315 | 28334 | 38 |
| UK-Br | GCGTTCTCCATTCTGGTTACT | RV | 28349 | 28369 | 39 |
| UK-Cr | GAATCTGAGGGTCCACCAAA | RV | 28322 | 28341 | 40 |
| UK-Dr | CTGCGTTCTCCATTCTGGTT | RV | 28352 | 28371 | 41 |
| UK-Er | GTAGTGCGTTGTTCGTTCTA | RV | 28188 | 28207 | 42 |
| UK-Fr | CTGGTTACTGCCAGTTGAAT | RV | 28338 | 28357 | 43 |
| UK-probeA | TGTCTCTAAATGGACCCCAAAATCAGC | FW | 28275 | 28301 | 44 |
| UK-probeB | GCTGATTTTGGGGTCCATTTAGAG | RV | 28278 | 28301 | 45 |
| UK-probeC | CTCTAAATGGACCCCAAAATCAGC | FW | 28278 | 28301 | 46 |

### C. Nucleic Acid Extraction

RNA is extracted from specimens using the NucleoSpin Dx Virus (Macherey Nagel.. ref. 740895.50). RNA extracted from 100 µl of original sample, is eluted in 100 µl of elution buffer.

**Table 20 : SARS-CoV-2 variant strains used to test primers and probes**

| **Origin** | **Variant** | **Clades** | **Pango lineages** | |
|---|---|---|---|---|
| France | CIBU-200132 | | | CIBU, Institut Pasteur, Paris |
| France | D614G | G | | NRC for Respiratory Viruses, Institut Pasteur, Paris |
| UK | 20I/501Y.V1 | GR | B.1.1.7 | NRC for Respiratory Viruses, Institut Pasteur, Paris |
| SA | 20H/501Y.V2 | GH | B.1.351 | NRC for Respiratory Viruses, Institut Pasteur, Paris |
| BR | 20J/501Y P1 | GR | P.1 | NRC for Respiratory Viruses, Institut Pasteur, Paris |

### D. Mix Preparation

All primers and probes described below were validated under the following conditions.

A variable volume of RNA was reverse transcribed using iScript Advanced cDNA synthesis kit for RT-qPCR (Bio-Rad) with 4µl of 5x iScript Advanced Reaction Mix and 1µl of iScript Advanced Reverse Transcriptase in a final volume of 20µl for 30 min 42°C, 5min 85°C in T100 Thermal Cycler (Bio-Rad)

Adjustments may be required for the use of other kits or other real-time PCR instruments.

All Assays used the same conditions. Primer and probe sequences, as well as optimized concentrations are shown in table below. A 20µl reaction was set up containing 2µl of RNA, 400nM of primers, and optionally 200nM of probe.

**Table 21: Simplex mixture**

| **Simplex Mix with SYBRGreen^{®} dye** | **Vol (µl)** | **[final]** |
|---|---|---|
| H₂O PPI | 6.4 | |
| SYBR green mix 2X | 10 | 1X |
| Forward Primer (10µM) | 0.8 | 0.4 µM |
| Reverse Primer (10µM) | 0.8 | 0.4 µM |
| **Final Volume** | **20.00** | |

**Table 22: Multiplex mixture**

| **Simplex Mix with a probe** | **Vol (µl)** | **[final]** |
|---|---|---|
| H2O PPI | 6 | |
| SYBR green mix 2X | 10 | 1X |
| Forward Primer (10µM) | 0.8 | 0.4 µM |
| Reverse Primer (10µM) | 0.8 | 0.4 µM |
| Probe (10µM) | 0.4 | 0.2 µM |
| **Final Volume** | **20.00** | |

### E. Controls

All primers and probes were tested by RT-qPCR with an RNA extract form HEK-293T (Hum Embryonic Kidney) human cell line or without RNA template (H₂O).

### F. Amplification cycles

**Table 23: Amplification cycles**

| | | | | |
|---|---|---|---|---|
| Denaturation | 95°C | 2 min | x1 | |
| Amplification | 95°C | 5 sec | X40 | Acquisition |
| | 60°C | 30 sec | | |
| Cooling | 40°C | 30 sec | x1 | |

### G. Analytical specificity of the primer pairs

Since the designed probes target the 3 nucleotides mutation, it was possible to select the best primer pairs on ancestral SARS-CoV-2 RNA (extracted from clinical sample CIBU-200132 strains) with a DNA intercalant dye before testing combinations of the best primer pairs with a probe (Figure 3).

16 combinations of primer pairs were tested including the following pairs:
- UK-As, UK-Ar,
- UK-Bs, UK-Br,
- UK-Cs, UK-Cr,
- UK-Ds, UK-Cr,
- UK-D2s, UK-Cr,
- UK-D2s, UK-Dr, UK-Es, UK-Er,
- UK-Fs, UK-Fr,
- UK-As, UK-Br,
- UK-As, UK-Cr,
- UK-Bs, UK-Ar,
- UK-Bs, UK-Cr,
- UK-Ds, UK-Ar,

The specificity of the primer pairs was assessed using serial 10-fold dilutions of the genomic SARS-CoV-2 and by analyzing the melt curve.

### H. Amplification efficiencies

Based on the best primer pairs, 16 combinations of primers and probe were tested including the following combinations:
- MUT-As, MUT-Ar, MUT-probeA
- MUT-Bs, MUT-Br, MUT-probeA
- MUT-Bs, MUT-Br, MUT-probeB
- MUT-Cs, MUT-Cr, MUT-probeA
- MUT-Ds, MUT-Dr, MUT-probeA
- MUT-Ds, MUT-Dr, MUT-probeB
- UK-As, UK-Br, UK-probeB
- UK-As, UK-Cr, UK-probeB
- UK-Bs, UK-Ar, UK-probeB
- UK-Bs, UK-Cr, UK-probeB
- UK-Ds, UK-Ar, UK-probeB

All combinations were tested on RNA extract from UK, SA or BR variant, ancestral SARS-CoV-2, and controls (HEK-293T and H₂O) (Figure 2 and 4).

The following quantification cycle (Cq) values were obtained:

**Table 24 : Cq values obtained with each primers-probe combination for RNA samples from UK variant vs ancestral SARS-CoV-2.**

| **Origin** | Copies | Cq UKAs-Br | Cq UKAs-Cr | Cq UKBs-Ar | Cq UKBs-Cr | Cq UKDs-Ar |
|---|---|---|---|---|---|---|
| | | UK-Probe B | UK-Probe B | UK-Probe B | UK-Probe B | UK-Probe B |
| France (ancestral) | 3.10⁵ | 0.00 | 0.00 | 0.00 | 0.00 | 0.00 |
| UK | 3.10⁵ | 29.28 | 29.15 | 29.14 | 28.90 | 28.97 |

No amplification was detected in controls or with RNA samples from SA or BR variants.

The amplification efficiency (E) was determined by plotting the mean Cq values against five different copy numbers (10-fold dilutions).

**Table 25 : Cq values obtained with each primers-probe combination for a determined copy number of UK RNA, the dilution range being used as the standard curve. (nd: not detected)**

| UK RNA range (Copy nb) | Cq | Cq | Cq | Cq | Cq |
|---|---|---|---|---|---|
| | UK As-Br | UK As-Cr | UK Bs-Ar | UK Bs-Cr | UK Ds-Ar |
| | UK-Probe B | UK-Probe B | UK-Probe B | UK-Probe B | UK-Probe B |
| 4.10⁵ | 27,41 | 27.38 | 27.74 | 27.63 | 27.88 |
| 4.10⁴ | 30,46 | 30.41 | 31.44 | 30.83 | 31.03 |
| 4.10³ | 33,67 | 34.11 | 35.11 | 33.56 | 34.31 |
| 4.10² | 37,37 | 37.03 | 37.48 | 36.98 | 37.12 |
| 40 | nd | 39.62 | nd | nd | 39.67 |

**Table 26 : Efficiency of the primers sets to detect specifically the UK variant.**

| **Primer sets (with UK-probeB probe)** | **Efficacity (%)** |
|---|---|
| UK As+Br | 100.5 |
| UK As+Cr | 107.2 |
| UK Bs+Ar | 103.2 |
| UK Bs+Cr | 108.6 |
| UK Ds+Cr | 104.2 |

Among the 16 combination of primers and probe tested, only the 5 following sets have an efficacy of at least 100%:
- UK-As, UK-Br, UK-probeB
- UK-As, UK-Cr, UK-probeB
- UK-Bs, UK-Ar, UK-probeB
- UK-Bs, UK-Cr, UK-probeB
- UK-Ds, UK-Ar, UK-probeB

Since these 5 primers and probe sets does not detect ancestral SARS-CoV-2 virus, SA or BR variants or controls, they enable the specific detection of the UK variant.

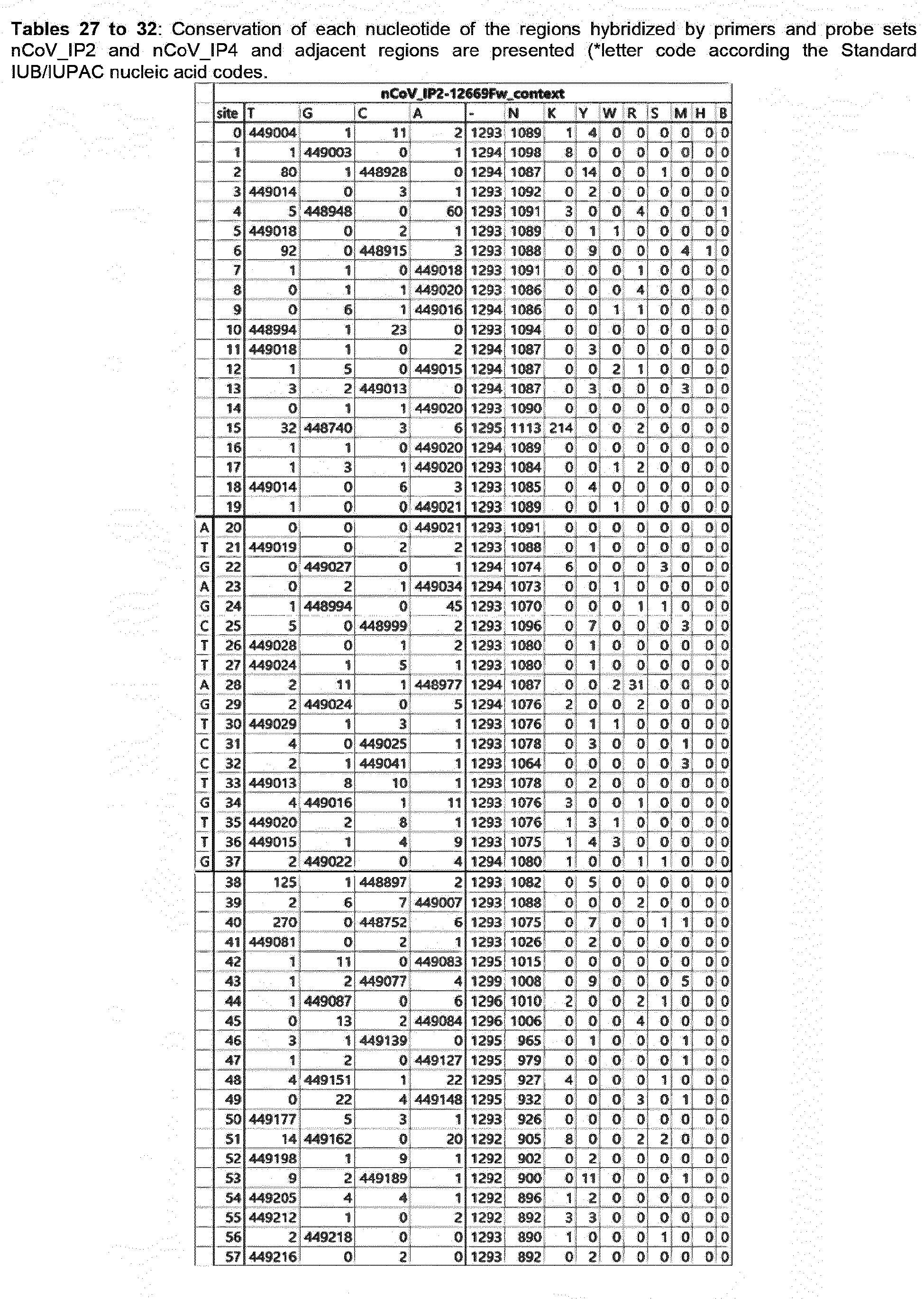

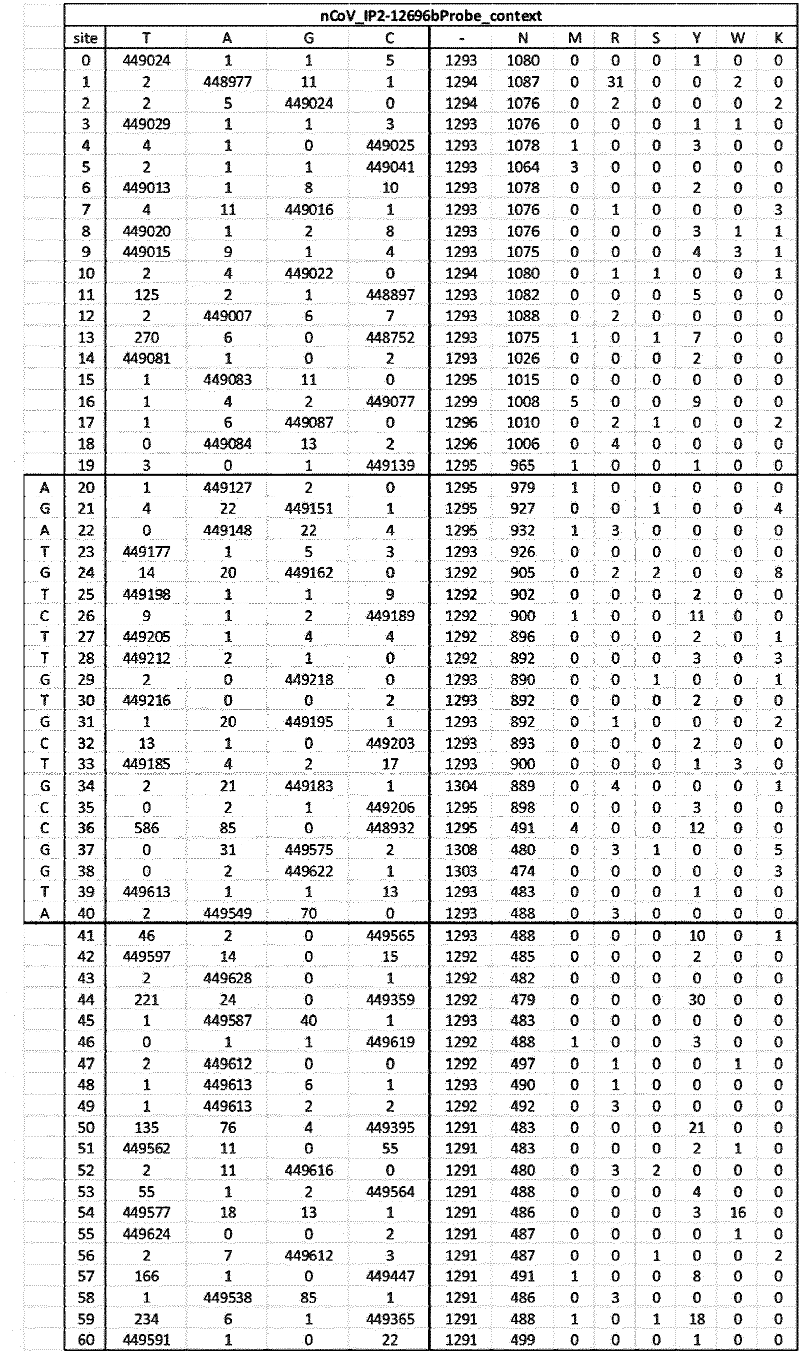

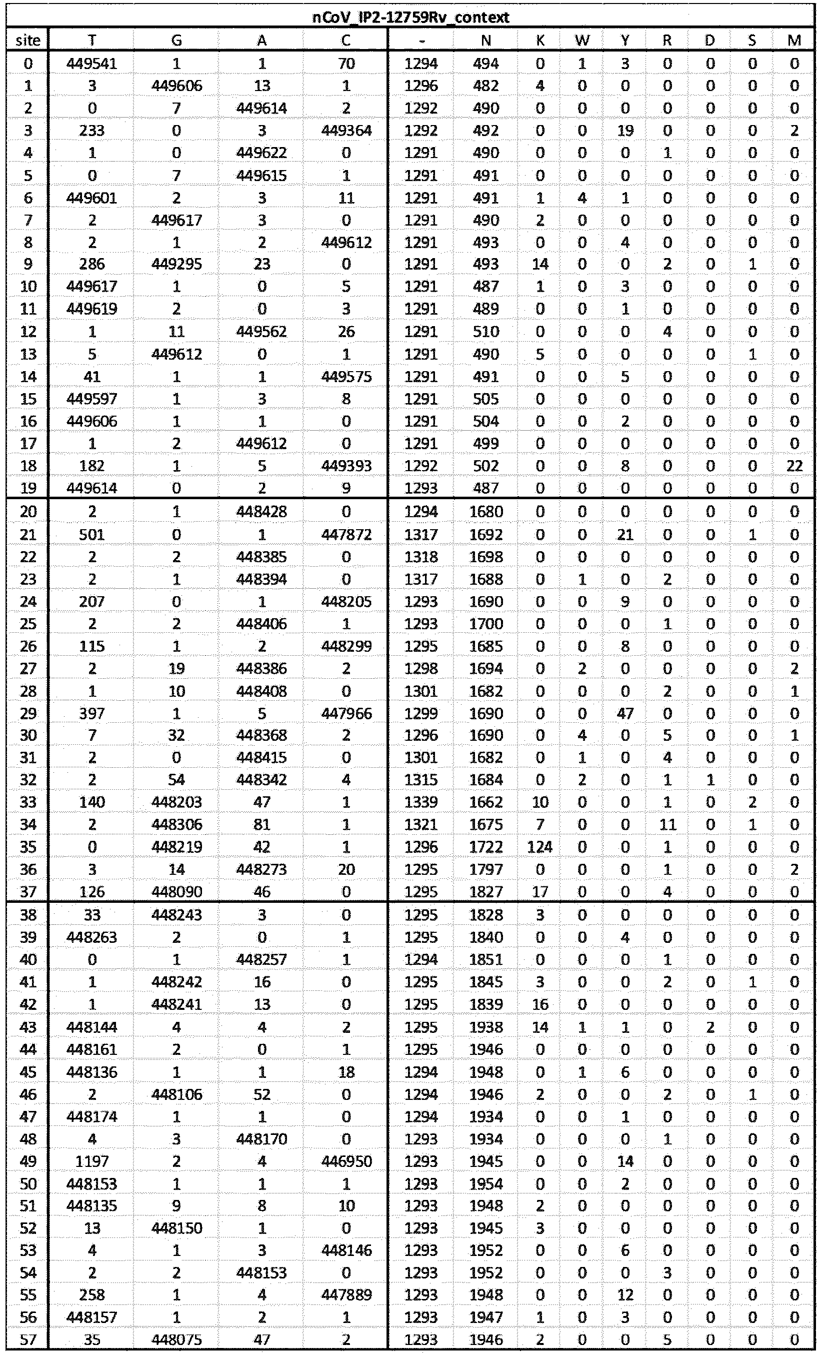

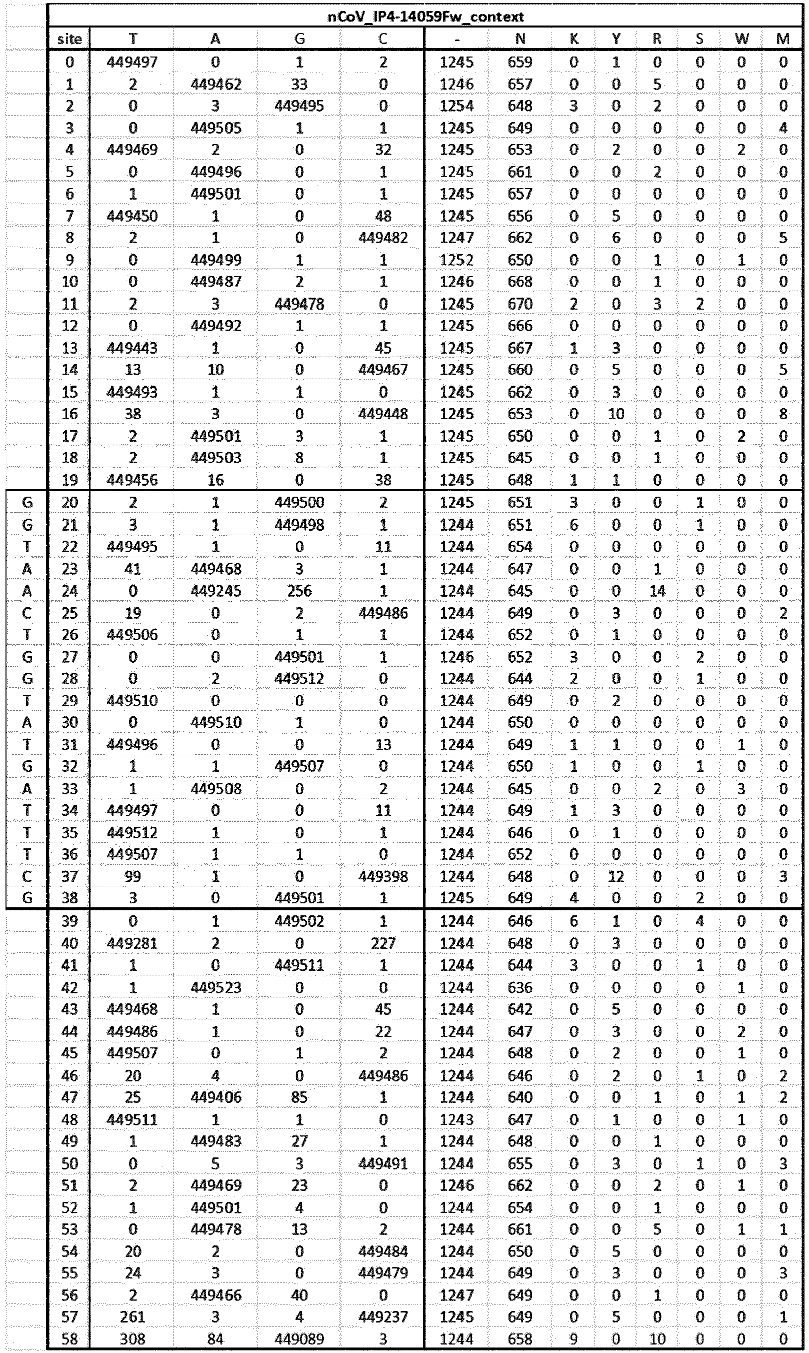

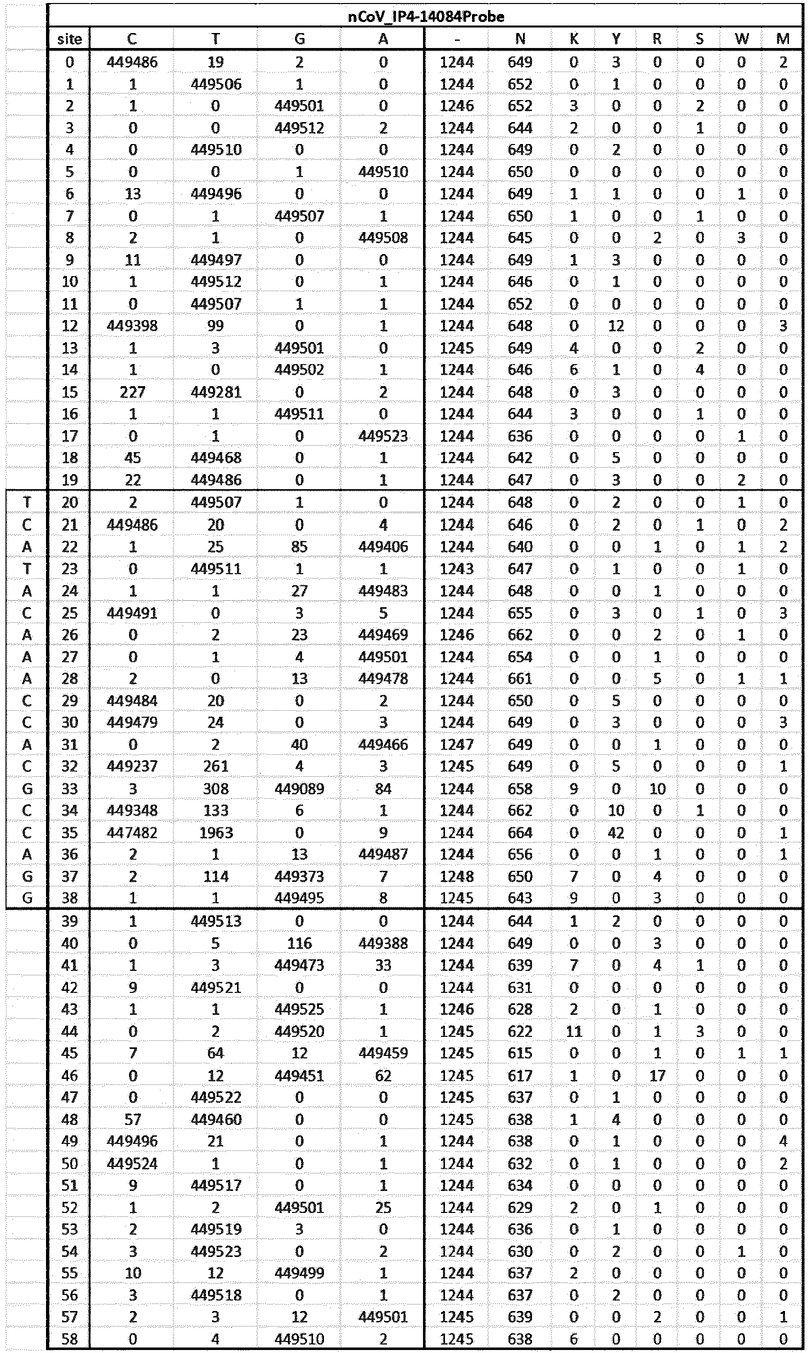

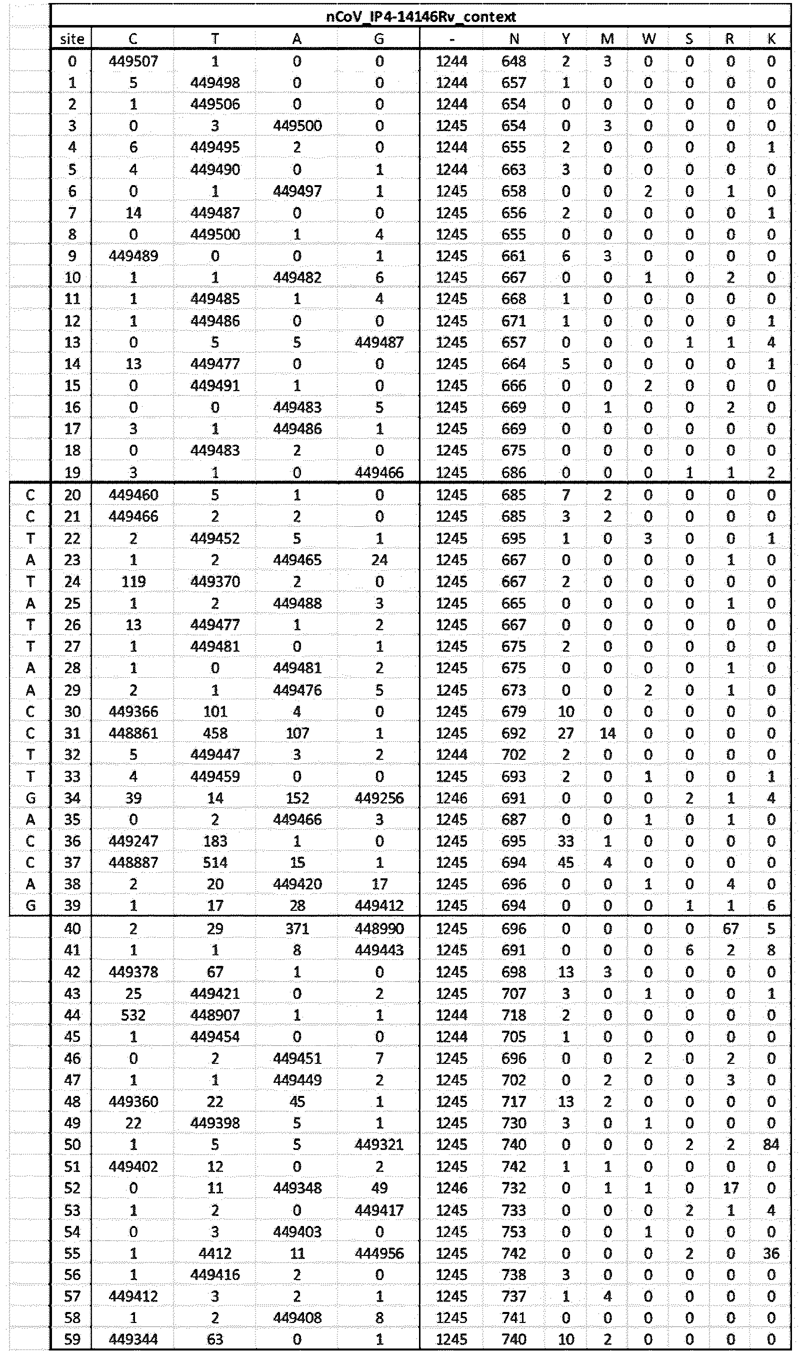

## Claims

1. A multiplex method for specific detection of the presence or absence of a SARS-CoV-2 RNA in a sample, comprising:
subjecting a sample to two simultaneous amplification reactions:
(A) a first reverse transcription reaction with a first SARS-CoV-2-specific reverse primer to generate a first cDNA copy of SARS-CoV-2 RNA in the sample; amplifying any resultant first cDNA with the first reverse primer and a first SARS-CoV-2 specific forward primer; and detecting any first amplified product with a first SARS-CoV-2 specific probe; wherein the first SARS-CoV-2 specific reverse primer hybridizes to the sequence: 5'-ACAACACAACAAAGGGAG-3' (SEQ ID NO: 11) and the RNA equivalent thereof; the first SARS-CoV-2 specific forward primer hybridizes to the sequence: 5'-CAACAGGACTAAGCTCAT-3' (SEQ ID NO: 10) and the first SARS-CoV-2 specific probe hybridizes to the sequence: 5'-TACCGGCAGCACAAGACATCT-3' (SEQ ID NO: 12) or the complement thereof; and
(B) a second reverse transcription reaction with a second SARS-CoV-2-specific reverse primer to generate a second cDNA copy of SARS-CoV-2 RNA in the sample; amplifying any resultant second cDNA with the second reverse primer and a second SARS-CoV-2 specific forward primer; and detecting any second amplified product with a second SARS-CoV-2 specific probe; wherein the second SARS-CoV-2 specific reverse primer hybridizes to the sequence: 5'-CCTATATTAACCTTGACCAG-3' (SEQ ID NO: 14) and the RNA equivalent thereof, the second SARS-CoV-2 specific forward primer hybridizes to the sequence: 5'-CGAAATCATACCAGTTACC-3' (SEQ ID NO: 13), and the second SARS-CoV-2 specific probe hybridizes to the sequence: 5'-CCTGGCGTGGTTTGTATGA-3' (SEQ ID NO: 15) or the complement thereof; and
wherein said SARS-CoV-2 is chosen from SARS-CoV-2 variants of isolate Wuhan-Hu-1 carrying mutations in the Spike protein predicted to increase viral particles binding affinity towards the human ACE2 receptor and/or reduce viral particles sensitivity to neutralizing antibodies.

2. The method of claim 1, wherein said variants carry one or more predicted mutations in the Spike protein selected from the group consisting of: E484K; K417N, N501Y, A570D, P681H, T716I, S982A, D1118H, del69/70 and del144/145.

3. The method of claim 1 or claim 2,
wherein the first SARS-CoV-2 specific reverse primer comprises the sequence 5'-CTCCCTTTGTTGTGTTGT-3' (SEQ ID NO: 2), and is no more than 21 bases in length; and
wherein the first SARS-CoV-2 specific forward primer comprises the sequence 5'-ATGAGCTTAGTCCTGTTG-3' (SEQ ID NO: 1), and is no more than 21 bases in length; and
wherein the first SARS-CoV-2 specific probe comprises the sequence 5'-AGATGTCTTGTGCTGCCGGTA-3' (SEQ ID NO: 3), or the complement thereof, and is no more than 24 bases in length.

4. The method of any one of claims 1 to 3,
wherein the second SARS-CoV-2 specific reverse primer comprises the sequence 5'- CTGGTCAAGGTTAATATAGG -3' (SEQ ID NO: 5), and is no more than 23 bases in length; and
wherein the second SARS-CoV-2 specific forward primer consists of or comprises the sequence 5'- GGTAACTGGTATGATTTCG -3' (SEQ ID NO: 4), and is no more than 22 bases in length; and
wherein the second SARS-CoV-2 specific probe consists of or comprises the sequence 5'- TCATACAAACCACGCCAGG -3' (SEQ ID NO: 6), or the complement thereof, and is no more than to 22 bases in length.

5. The method of any of claims 1 to 4, wherein the method is an RT-PCR method, preferably quantitative reverse-transcriptase PCR (QRT-PCR).

6. The method of any of claims 1 to 5, wherein the probe is labelled with 6-carboxy-fluorescein (6FAM) or hexachloro-6-carboxy-fluorescein (HEX) at the 5' end; and/or black hole quencher 1 (BHQ1) at the 3' end.

7. The method of any of claims 1 to 6, wherein the sample is an environmental sample or a biological sample, preferably a body fluid sample, more preferably oral or respiratory tract secretions.

## Patentansprüche

1. Multiplexverfahren zum spezifischen Nachweis des Vorhandenseins oder Nichtvorhandenseins von einer SARS-CoV-2-RNA in einer Probe, umfassend:
Unterziehen einer Probe zwei gleichzeitigen Amplifikationsreaktionen:
(A) einer ersten reversen Transkriptionsreaktion mit einem ersten SARS-CoV-2-spezifischen Rückwärtsprimer, um eine erste cDNA-Kopie von einer SARS-CoV-2-RNA in der Probe zu erzeugen; Amplifizieren jeder resultierenden ersten cDNA mit dem ersten Rückwärtsprimer und einem ersten SARS-CoV-2-spezifischen Vorwärtsprimer; und Nachweisen von jedem ersten amplifizierten Produkt mit einer ersten SARS-CoV-2-spezifische Sonde; wobei der erste SARS-CoV-2-spezifische Rückwärtsprimer zu der Sequenz 5'-ACAACACAACAAAGGGAG-3' (SEQ ID NO: 11) und dem RNA-Äquivalent davon hybridisiert; der erste SARS-CoV-2-spezifische Vorwärtsprimer zu der Sequenz 5'-CAACAGGACTAAGCTCAT-3' (SEQ ID NO: 10) hybridisiert und die erste SARS-CoV-2-spezifische Sonde zu der Sequenz 5'-TACCGGCAGCACAAGACATCT-3' (SEQ ID NO: 12) oder dem Komplement davon hybridisiert; und
(B) einer zweiten reversen Transkriptionsreaktion mit einem zweiten SARS-CoV-2-spezifischen Rückwärtsprimer, um eine zweite cDNA-Kopie der SARS-CoV-2-RNA in der Probe zu erzeugen; Amplifizieren jeder resultierenden zweiten cDNA mit dem zweiten Rückwärtsprimer und einem zweiten SARS-CoV-2-spezifischen Vorwärtsprimer; und Nachweisen von jedem zweiten amplifizierten Produkt mit einer zweiten SARS-CoV-2-spezifischen Sonde; wobei der zweite SARS-CoV-2-spezifische Rückwärtsprimer zu der Sequenz 5'-CCTATATTAACCTTGACCAG-3' (SEQ ID NO: 14) und dem RNA-Äquivalent davon hybridisiert, der zweite SARS-CoV-2-spezifische Vorwärtsprimer zu der Sequenz 5'-CGAAATCATACCAGTTACC-3' (SEQ ID NO: 13) hybridisiert, und die zweite SARS-CoV-2 spezifische Sonde zu der Sequenz 5'-CCTGGCGTGGTTTGTATGA-3' (SEQ ID NO: 15) oder dem Komplement davon hybridisiert; und
wobei das SARS-CoV-2 ausgewählt ist aus SARS-CoV-2-Varianten eines Wuhan-Hu-1-Isolats, das Mutationen in dem Spike-Protein trägt, von denen vorhergesagt wird, dass sie die Bindungsaffinität von viralen Partikeln gegenüber dem menschlichen ACE2-Rezeptor erhöhen und/oder eine Empfindlichkeit von viralen Partikeln gegenüber neutralisierenden Antikörpern reduzieren.

2. Verfahren nach Anspruch 1, wobei die Varianten eine oder mehrere vorhergesagte Mutationen in dem Spike-Protein tragen, die ausgewählt sind aus der Gruppe bestehend aus: E484K; K417N, N501Y, A570D, P681H, T716I, S982A, D1118H, del69/70 und del144/145.

3. Verfahren nach Anspruch 1 oder Anspruch 2,
wobei der erste SARS-CoV-2-spezifische Rückwärtsprimer die Sequenz 5'-CTCCCTTTGTTGTGTTGT-3' (SEQ ID NO: 2) umfasst, und nicht mehr als 21 Basen lang ist; und
wobei der erste SARS-CoV-2-spezifische Vorwärtsprimer die Sequenz 5'-ATGAGCTTAGTCCTGTTG-3' (SEQ ID NO: 1) umfasst, und nicht mehr als 21 Basen lang ist; und
wobei die erste SARS-CoV-2-spezifische Sonde die Sequenz 5'-AGATGTCTTGTGCTGCCGGTA-3' (SEQ ID NO: 3) oder das Komplement davon umfasst, und nicht mehr als 24 Basen lang ist.

4. Verfahren nach einem der Ansprüche 1 bis 3,
wobei der zweite SARS-CoV-2-spezifische Rückwärtsprimer die Sequenz 5'-CTGGTCAAGGTTAATATAGG-3' (SEQ ID NO: 5) umfasst, und nicht mehr als 23 Basen lang ist; und
wobei der zweite SARS-CoV-2-spezifische Vorwärtsprimer aus der Sequenz 5'-GGTAACTGGTATGATTTCG-3' (SEQ ID NO: 4) besteht oder diese umfasst, und nicht mehr als 22 Basen lang sind; und
wobei die zweite SARS-CoV-2-spezifische Sonde aus der Sequenz 5'-TCATACAAACCACGCCAGG-3' (SEQ ID NO: 6) oder dem Komplement davon besteht oder diese umfasst, und nicht mehr als 22 Basen lang ist.

5. Verfahren nach einem der Ansprüche 1 bis 4, wobei das Verfahren eine RT-PCR-Methode, vorzugsweise quantitative Reverse-Transkriptase-PCR (QRT-PCR) ist.

6. Verfahren nach einem der Ansprüche 1 bis 5, wobei die Sonde mit 6-Carboxy-fluorescein (6FAM) oder Hexachloro-6-carboxy-fluorescein (HEX) an dem 5'-Ende und/oder mit Black-Hole-Quencher 1 (BHQ1) am 3'-Ende markiert ist.

7. Verfahren nach einem der Ansprüche 1 bis 6, wobei es sich bei der Probe um eine Umweltprobe oder eine biologische Probe, vorzugsweise eine Körperflüssigkeitsprobe, besonders bevorzugt Mund- oder Atemwegssekrete, handelt

## Revendications

1. Procédé multiplex de détection spécifique de la présence ou de l'absence d'un ARN de SARS-CoV-2 dans un échantillon, comprenant :
l'exposition d'un échantillon à deux réactions d'amplification simultanées :
(A) une première réaction de transcription inverse avec une première amorce inverse spécifique à SARS-CoV-2 pour générer une première copie d'ADNc de l'ARN de SARS-CoV-2 dans l'échantillon ; l'amplification d'un quelconque premier ADNc résultant avec la première amorce inverse et une première amorce directe spécifique à SARS-CoV-2 ; et la détection d'un quelconque premier produit amplifié avec une première sonde spécifique à SARS-CoV-2 ; dans lequel la première amorce inverse spécifique à SARS-CoV-2 s'hybride à la séquence : 5'-ACAACACAACAAAGGGAG-3' (SEQ ID NO : 11) et à son équivalent ARN ; la première amorce directe spécifique à SARS-CoV-2 s'hybride à la séquence : 5'-CAACAGGACTAAGCTCAT-3' (SEQ ID NO : 10) et la première sonde spécifique à SARS-CoV-2 s'hybride à la séquence : 5'-TACCGGCAGCACAAGACATCT-3' (SEQ ID NO : 12) ou à son complément ; et
(B) une deuxième réaction de transcription inverse avec une deuxième amorce inverse spécifique à SARS-CoV-2 pour générer une deuxième copie d'ADNc de l'ARN de SARS-CoV-2 dans l'échantillon ; l'amplification d'un quelconque deuxième ADNc résultant avec la deuxième amorce inverse et une deuxième amorce directe spécifique à SARS-CoV-2 ; et la détection d'un quelconque deuxième produit amplifié avec une deuxième sonde spécifique à SARS-CoV-2 ; dans lequel la deuxième amorce inverse spécifique à SARS-CoV-2 s'hybride à la séquence : 5'-CCTATATTAACCTTGACCAG-3' (SEQ ID NO: 14) et à son équivalent ARN, la deuxième amorce directe spécifique à SARS-CoV-2 s'hybride à la séquence : 5'-CGAAATCATACCAGTTACC-3' (SEQ ID NO: 13), et la deuxième sonde spécifique à SARS-CoV-2 s'hybride à la séquence : 5'-CCTGGCGTGGTTTGTATGA-3' (SEQ ID NO : 15) ou à son complément ; et
dans lequel ledit SARS-CoV-2 est choisi parmi des variants de SARS-CoV-2 de l'isolat Wuhan-Hu-1 portant des mutations dans la protéine Spike supposées augmenter l'affinité de liaison des particules virales vis-à-vis du récepteur ACE2 humain et/ou réduire la sensibilité des particules virales aux anticorps neutralisants.

2. Procédé selon la revendication 1, dans lequel lesdits variants portent une ou plusieurs mutations prédites dans la protéine Spike sélectionnées dans le groupe constitué de : E484K ; K417N, N501Y, A570D, P681H, T716I, S982A, D1118H, del69/70 et del144/145.

3. Procédé selon la revendication 1 ou la revendication 2,
dans lequel la première amorce inverse spécifique à SARS-CoV-2 comprend la séquence 5'-CTCCCTTTGTTGTGTTGT-3' (SEQ ID NO : 2) et n'a pas plus de 21 bases de longueur ; et
dans lequel la première amorce directe spécifique à SARS-CoV-2 comprend la séquence 5'-ATGAGCTTAGTCCTGTTG-3' (SEQ ID NO : 1) et n'a pas plus de 21 bases de longueur ; et
dans lequel la première sonde spécifique à SARS-CoV-2 comprend la séquence 5'-AGATGTTCTTGTGCTGCCGGTA-3' (SEQ ID NO : 3), ou son complément, et n'a pas plus de 24 bases de longueur.

4. Procédé selon l'une quelconque des revendications 1 à 3,
dans lequel la deuxième amorce inverse spécifique à SARS-CoV-2 comprend la séquence 5'-CTGGTCAAGGTTAATATAGG-3' (SEQ ID NO : 5), et n'a pas plus de 23 bases de longueur ; et
dans lequel la deuxième amorce directe spécifique à SARS-CoV-2 est constituée de ou comprend la séquence 5'-GGTAACTGGTATGATTTCG-3' (SEQ ID NO : 4), et n'a pas plus de 22 bases de longueur ; et
dans lequel la deuxième sonde spécifique à SARS-CoV-2 est constituée de ou comprend la séquence 5'-TCATACAAACCACGCCAGG-3' (SEQ ID NO : 6), ou son complément, et n'a pas plus de 22 bases de longueur.

5. Procédé selon l'une quelconque des revendications 1 à 4, dans lequel le procédé est un procédé RT-PCR, de préférence une PCR quantitative à transcriptase inverse (QRT-PCR).

6. Procédé selon l'une quelconque des revendications 1 à 5, dans lequel la sonde est marquée d'une 6-carboxy-fluorescéine (6FAM) ou d'une hexachloro-6-carboxy-fluorescéine (HEX) à l'extrémité 5' ; et/ou d'un extincteur de luminescence de trou noir 1 (BHQ1) à l'extrémité 3'.

7. Procédé selon l'une quelconque des revendications 1 à 6, dans lequel l'échantillon est un échantillon environnemental ou un échantillon biologique, de préférence un échantillon de fluide corporel, plus préférablement des sécrétions buccales ou des voies respiratoires.
